# EUROPEAN PATENT SPECIFICATION

(11) **EP 2 274 007 B1**
(45) Date of publication and mention of the grant of the patent: **14.12.2016**
(21) Application number: 09720014.1
(22) Date of filing: 10.03.2009
(51) Int. Cl.: A61K 31/52, A61K 39/395, A61P 25/28

(54) **MODULATION OF BLOOD BRAIN BARRIER PERMEABILITY**
MODULATION DER DURCHLÄSSIGKEIT DER BLUT-HIRN-SCHRANKE
MODULATION DE LA PERMÉABILITÉ D'UNE BARRIÈRE HÉMATOENCÉPHALIQUE

(30) Priority: 17.03.2008 US 37145; 10.03.2008 US 35250
(43) Date of publication of application: 19.01.2011
(73) Proprietor: Cornell University, Ithaca, NY 14850 (US)
(72) Inventor: BYNOE, Margaret, S., Ithaca NY 14850 (US)
(74) Representative: Potter Clarkson LLP
(86) International application number: PCT/US2009/036671
(87) International publication number: WO 2009/114533

(56) References cited:
- WO-A2-00/72799
- WO-A2-01/13935
- US-A1- 2001 027 196
- US-A1- 2006 198 821
- US-A1- 2008 025 959
- US-A1- 2008 027 081
- US-A1- 2008 027 082
- GRANT GERALD A ET AL: "Adenosine-induced modulation of excitatory amino acid transport across isolated brain arterioles", JOURNAL OF NEUROSURGERY, AMERICAN ASSOCIATION OF NEUROLOGICAL SURGEONS, US, vol. 98, no. 3, 1 March 2003 (2003-03-01), pages 554-560, XP009157119, ISSN: 0022-3085
- S. TSUTSUI: "A1 Adenosine Receptor Upregulation and Activation Attenuates Neuroinflammation and Demyelination in a Model of Multiple Sclerosis", JOURNAL OF NEUROSCIENCE, vol. 24, no. 6, 11 February 2004 (2004-02-11), pages 1521-1529, XP55022992, ISSN: 0270-6474, DOI: 10.1523/JNEUROSCI.4271-03.2004
- MAYHAN W G: "Role of nitric oxide in histamine-induced increases in permeability of the blood-brain barrier", BRAIN RESEARCH 1996 NL LNKD- DOI:10.1016/S0006-8993(96)01021-9, vol. 743, no. 1-2, 1996, pages 70-76, XP002672330, ISSN: 0006-8993
- AL MOUTAERY K ET AL: "Caffeine impairs short-term neurological outcome after concussive head injury in rats", NEUROSURGERY 20030901 US, vol. 53, no. 3, 1 September 2003 (2003-09-01), pages 704-712, XP009157117, ISSN: 0148-396X
- MARLOES P. SCHADDELEE ET AL: "Functional role of adenosine receptor subtypes in the regulation of blood-brain barrier permeability: possible implications for the design of synthetic adenosine derivatives", EUROPEAN JOURNAL OF PHARMACEUTICAL SCIENCES, vol. 19, no. 1, 1 May 2003 (2003-05-01), pages 13-22, XP55022947, ISSN: 0928-0987, DOI: 10.1016/S0928-0987(03)00034-4
- LAURA AIRAS ET AL: "Mechanism of action of IFN-beta in the treatment of multiple sclerosis: a special reference to CD73 and adenosine", NEW YORK ACADEMY OF SCIENCES. ANNALS, WILEY-BLACKWELL PUBLISHING, INC, US, vol. 1110, 27 September 2007 (2007-09-27), pages 641-648, XP008131944, ISSN: 0077-8923, DOI: 10.1196/ANNALS.1423.067
- VICTORIA I. INGLIS ET AL: "Neutrophils both reduce and increase permeability in a cell culture model of the blood-brain barrier", BRAIN RESEARCH, vol. 998, no. 2, 1 February 2004 (2004-02-01), pages 218-229, XP55022985, ISSN: 0006-8993, DOI: 10.1016/j.brainres.2003.11.031
- M Bynoe: "CD73-deficient mice are resistant to experimental autoimmune encephalomyelitis", The Journal of Immunology, 2007, 178, 130.30, 1 April 2007 (2007-04-01), page S233, XP055171046, Retrieved from the Internet: URL:http://jimmunol.org/cgi/content/meetin g_abstract/178/MeetingAbstracts/S233-c [retrieved on 2015-02-20]
- J. H. Mills ET AL: "CD73 is required for efficient entry of lymphocytes into the central nervous system during experimental autoimmune encephalomyelitis", Proceedings of the National Academy of Sciences, vol. 105, no. 27, 8 July 2008 (2008-07-08) , pages 9325-9330, XP055171083, ISSN: 0027-8424, DOI: 10.1073/pnas.0711175105
- Linda F. Thompson ET AL: "Regulation of Leukocyte Migration Across Endothelial Barriers by ECTO-5'-Nucleotidase-Generated Adenosine", Nucleosides, Nucleotides & Nucleic Acids, vol. 27, no. 6-7, 23 July 2008 (2008-07-23), pages 755-760, XP055171090, ISSN: 1525-7770, DOI: 10.1080/15257770802145678
- Marc-André Bellavance ET AL: "Recent Advances in Blood-Brain Barrier Disruption as a CNS Delivery Strategy", The AAPS Journal, vol. 10, no. 1, 1 March 2008 (2008-03-01), pages 166-177, XP055229662, DOI: 10.1208/s12248-008-9018-7
- JACKSON SADHANA ET AL: "The effect of regadenoson-induced transient disruption of the blood-brain barrier on temozolomide delivery to normal rat brain", JOURNAL OF NEURO-ONCOLOGY, KLUWER, BOSTON, US, vol. 126, no. 3, 1 December 2015 (2015-12-01), pages 433-439, XP035616326, ISSN: 0167-594X, DOI: 10.1007/S11060-015-1998-4 [retrieved on 2015-12-01]

## Description

### FIELD OF THE INVENTION

The present invention relates to modulation of blood brain barrier permeability.

### BACKGROUND OF THE INVENTION

The barriers to blood entering the central nervous system ("CNS") are herein collectively referred to as the blood brain barrier ("BBB"). The BBB is a tremendously tight-knit layer of endothelial cells that coats 400 miles of capillaries and blood vessels in the brain (Ransohoff et al., "Three or More Routes for Leukocyte Migration Into the Central Nervous System," Nature Rev. Immun. 3:569-581 (2003)). The nearly impermeable junctions between BBB cells are formed by the interdigitation of about 20 different types of proteins. Molecules must enter a BBB cell through membrane-embedded protein transporters or by slipping directly through its waxy outer membrane. Once inside, foreign compounds must avoid a high concentration of metabolic enzymes and a variety of promiscuous protein pumps primed to eliminate foreign substances. Having avoided these obstacles, foreign molecules must then pass through the inner membrane of a BBB cell to finally reach the brain. These elaborate defenses allow the BBB to sequester the brain from potential harm, but the BBB also obstructs delivery of neurological drugs to a site of disease in the brain. Researchers in academia and the biotech and pharmaceutical industries are learning to bypass the BBB or allow it to let potential drugs into the brain. They are designing small drugs that can passively diffuse through the BBB or travel on nutrient transporters to get inside the brain. Others are attaching potential therapeutics designed so that the brain will unwittingly engulf them.

The capillaries that supply blood to the tissues of the brain constitute the blood brain barrier (Goldstein et al., "The Blood-Brain Barrier," Scientific American 255:74-83(1986); Pardridge, "Receptor-Mediated Peptide Transport Through the Blood-Brain Barrier," Endocrin. Rev. 7:314-330(1986)). The endothelial cells which form the brain capillaries are different from those found in other tissues in the body. Brain capillary endothelial cells are joined together by tight intercellular junctions which form a continuous wall against the passive diffusion of molecules from the blood to the brain and other parts of the CNS. These cells are also different in that they have few pinocytic vesicles which in other tissues allow somewhat unselective transport across the capillary wall. Also lacking are continuous gaps or channels running between the cells which would allow unrestricted passage.

The blood-brain barrier functions to ensure that the environment of the brain is constantly controlled. The levels of various substances in the blood, such as hormones, amino acids, and ions, undergo frequent small fluctuations which can be brought about by activities such as eating and exercise (Goldstein et al., "The Blood-Brain Barrier," Scientific American 255:74-83(1986); Pardridge, "Receptor-Mediated Peptide Transport Through the Blood-Brain Barrier," Endocrin. Rev. 7:314-330(1986)). If the brain was not protected by the blood brain barrier from these variations in serum composition, the result could be uncontrolled neural activity.

The isolation of the brain from the bloodstream is not complete. If this were the case, the brain would be unable to function properly due to a lack of nutrients and because of the need to exchange chemicals with the rest of the body. The presence of specific transport systems within the capillary endothelial cells assures that the brain receives, in a controlled manner, all of the compounds required for normal growth and function. In many instances, these transport systems consist of membrane-associated proteins, which selectively bind and transport certain molecules across the barrier membranes. These transporter proteins are known as solute carrier transporters.

Although it is believed that the BBB serves a protective function under normal conditions by protecting the CNS from exposure to potentially toxic compounds, in CNS disease, the BBB may thwart therapeutic efforts by hindering the entry of therapeutic compounds into the CNS. For example, although many bacterial and fungal infections may be readily treated where the site of the infection is outside the CNS, such infections in the CNS are often very dangerous and very difficult to treat due to the inability to deliver effective doses of drugs to the site of the infection. Similarly, the action of the BBB makes treatment of cancer of the brain more difficult than treatment of cancers located outside the CNS. Even where it may be possible to deliver an effective dose of drug into the CNS by administering very large amounts of drug outside of the CNS, the drug levels outside the CNS (such as in the blood) are then often so high as to reach toxic levels deleterious to the kidneys, liver, and other vital organs. Accordingly, there is need in the art for methods to improve the delivery of compounds into the CNS.

In addition, patients suffering from edema, brain traumas, stroke and multiple sclerosis exhibit a breakdown of the BBB near the site of primary insults. The level of breakdown can have profound effects on the clinical outcome of these diseases. For instance, the degree of BBB breakdown in patients suffering from multiple sclerosis ("MS") is correlated to the severity of the disease. It has been shown using Magnetic Resonance Imaging ("MRI") that, when a person is undergoing an MS "attack," the blood-brain barrier has broken down in a section of the brain or spinal cord, allowing white blood cells called T lymphocytes to cross over and destroy the myelin.

Despite the importance of this barrier, very little is known about the molecular mechanisms controlling the integrity and/or permeability of the BBB. Thus, there remains a considerable need for compositions and methods that facilitate such research and especially for diagnostic and/or therapeutic applications.

The present invention is directed to overcoming these and other deficiencies in the art.

### SUMMARY OF THE INVENTION

The invention is defined by the claims.

The present invention relates to an adenosine receptor agonist and/or an A1 adenosine receptor antagonist for use in increasing blood brain barrier permeability in a subject, wherein the subject is one who would benefit from increased blood brain barrier permeability and wherein the subject is not subjected to a treatment which increases adenosine level. The adenosine receptor agonist and/or an A1 adenosine receptor antagonist, modulates adenosine receptors under conditions effective to increase blood brain barrier permeability in the subject.

The present invention also relates to an A2A adenosine receptor antagonist for use in decreasing blood brain barrier permeability in a subject, wherein the subject is one who would benefit from decreased blood brain permeability The A2A adenosine receptor antagonist modulates adenosine receptors under conditions effective to decrease blood brain barrier permeability in the subject.

Also disclosed is a method of treating a subject for a disorder or condition of the central nervous system. This method involves selecting a subject with the disorder or condition of the central nervous system and providing a therapeutic effective to treat the disorder or condition of the central nervous system. A blood brain barrier permeabilizing agent, where the agent increases adenosine level and/or bioavailability, modulates adenosine receptors, and/or increases CD73 level and/or activity, is provided. The therapeutic and the blood brain barrier permeabilizing agent are administered to the selected subject under conditions effective for the therapeutic to pass across the blood brain barrier and treat the disorder or condition.

Also disclosed is a method of screening compounds effective in increasing blood brain barrier permeability. This method involves providing a modified animal with reduced CD73 expression levels, reduced adenosine expression levels, and/or modulated adenosine receptor activity compared to an unmodified animal. One or more candidate compounds are also provided, and the one or more candidate compounds are administered to the modified animal. It is then evaluated whether the one or more candidate compounds increases adenosine level and/or bioavailability, modulates adenosine receptors, and/or increases CD73 level and/or activity. Those candidate compounds which increase adenosine level or bioavailability, modulate adenosine receptors, and/or increase CD73 level and/or activity in the modified animal are then identified as being potentially effective in increasing blood brain barrier permeability.

Also disclosed is a pharmaceutical agent. This pharmaceutical agent has a therapeutic effective to treat the disorder or condition of the central nervous system and a blood brain barrier permeabilizing agent. That agent increases adenosine level and/or bioavailability, modulates adenosine receptors, and/or increases CD73 level and/or activity.

The agents of the present invention provide for an improved treatment of subjects with disorders affecting the blood brain barrier. In addition, the present invention provides agents for use in improved methods of controlling the blood brain barrier to enhance therapeutic treatment of such patients.

### BRIEF DESCRIPTION OF THE DRAWINGS

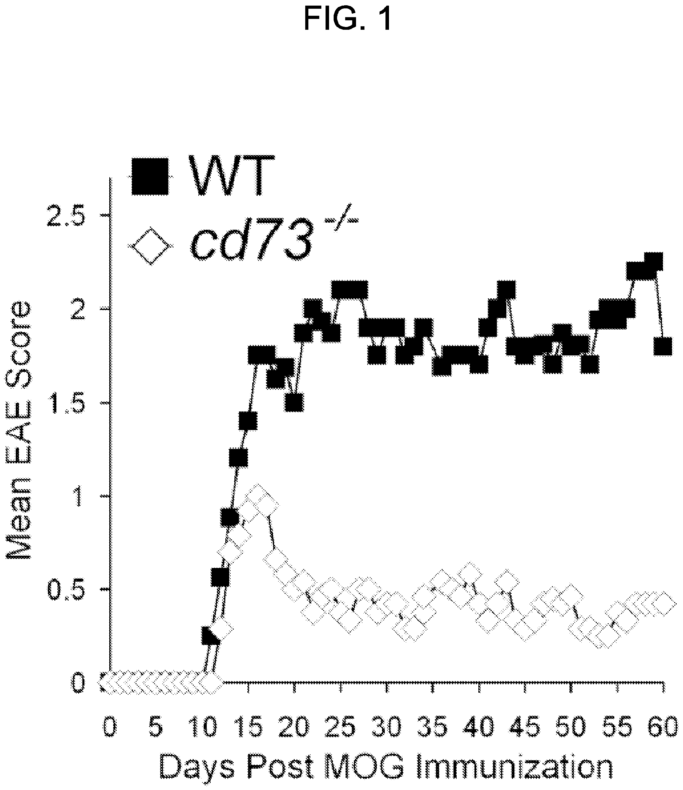

Figure 1 shows a graph demonstrating *cd73*^{*-*/*-*} mice are resistant to Experimental Autoimmune Encephalomyelitis ("EAE"). EAE was induced, disease activity was monitored daily, and the mean EAE score was calculated for *cd73*^{*-*/*-*} (open diamonds, n=11) and wild type (*cd73*^{+/+}) (closed squares, n=13) mice. The results shown are representative of 11 separate experiments.

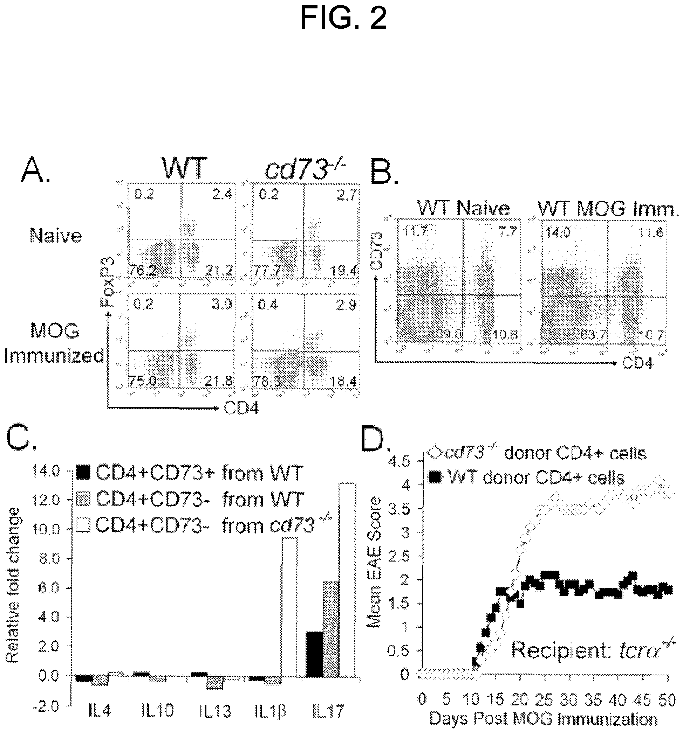

Figures 2A-D show cd73^{-/-} T cells produce elevated levels of IL-1β and IL-17 and mediate EAE susceptibility when transferred to *cd73*^{+/+}*tcr*α^{-/-} mice. Figure 2A shows the CD4 and FoxP3 expression measured on splenocytes from naive and day 13 post-EAE induced *cd73*^{-/-} and wild type mice. Figure 2B shows splenocytes from naive and day 13 post-MOG immunized wild type mice which were analyzed for CD4 and CD73 cell surface expression by flow cytometry. Figure 2C shows sorted cells from immunized wild type or *cd73*^{-/-} mice which were cultured with 1x10⁴ irradiated splenocytes and 0 or 10µM MOG peptide. Supernatants were taken at 18 hours and run on a cytokine Bio-plex assay. Results represent the fold change in cytokine levels between the 0 and 10µM MOG peptide groups. Samples were pooled from 4 mice and are representative of one out of three similar experiments. Figure 2D shows CD4⁺ T cells from the spleen and lymph nodes from MOG immunized *cd73*^{-/-} (open diamonds, n=5) or wild type (closed squares, n=5) mice which were adoptively transferred into T cell deficient *cd73*^{+/+}*tcrα*^{-/-} mice. EAE was induced and disease progression was monitored daily. Results are representative of two separate experiments.

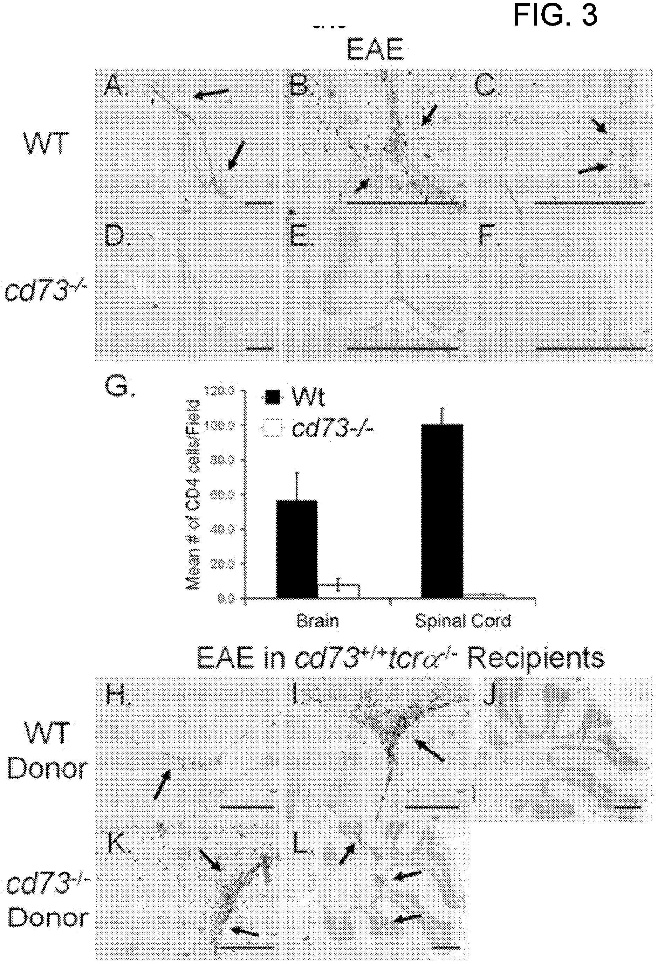

Figure 3A-L show cd73^{-/-} mice which display little or no CNS lymphocyte infiltration following EAE induction; donor *cd73*^{-/-} T cells infiltrate the CNS of *cd73*^{+/+}*tcrα*^{-/-} recipient mice following EAE induction. Frozen tissue sections from day 13 post-EAE induction wild type (Figures 3A-C) and *cd73*^{-/-} (Figures 3D-F) mice were labeled with a CD4 antibody. Figures 3G shows the mean number of CD4⁺ infiltrating lymphocytes in the brain and spinal cord quantified per field in frozen tissue sections from day 13 post-EAE induction wild type and *cd73*^{-/-} mice. Eight anatomically similar fields per brain and 4 fields per spinal cord per mouse were analyzed at 10x magnification (n=5 mice/group). Error bars represent the standard error of the mean. Figures 3H-L show frozen tissue sections of hippocampus (Fig. 3H, 3I, and 3K) and cerebellum (Fig.3J and 3L) labeled with a CD4 antibody from EAE-induced *tcrα*^{-/-} mice that received CD4⁺ cells from wild type (Fig. 3H-J) or *cd73*^{-/-} (Fig. 3K-L) mice at day 12 (Fig. 3K), 18 (Fig. 3H and 3L), or 22 (Fig. 3I and 3J) post-EAE induction. Immunoreactivity was detected with HRP anti-rat Ig plus AEC (red) against a hemotoxylin stained nuclear background (blue). Arrows indicate sites of lymphocyte infiltration. Scale bars represent 500µm.

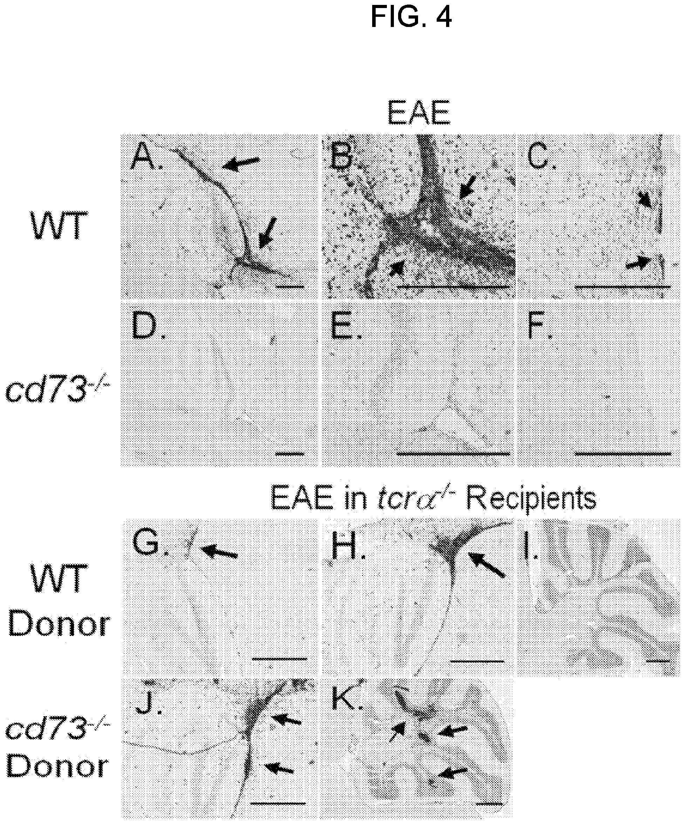

Figures 4A-K show *cd73*^{-/-} mice which display little or no CNS lymphocyte infiltration following EAE induction; *cd73*^{-/-} T cells infiltrate the CNS after transfer to *cd73*^{+/+}*tcrα*^{-/-} mice and EAE induction. Frozen tissue sections from day 13 post-EAE induction wild type (Fig. 4A-C) and *cd73*^{-/-} (Fig. 4D-F) mice were labeled with a CD45 antibody. Frozen tissue sections of hippocampus (Fig. 4G, 4H, and 4J) and cerebellum (Fig. 4I and 4K) labeled with a CD45 antibody from EAE-induced *tcrα*^{-/-} mice that received CD4⁺ cells from wild type (Fig. 4G-I) or *cd73*^{-/-} (Fig. 4J-K) mice at day 12 (Fig. 4J), day 18 (Fig. 4G and 4K), or day 22 (Fig. 4H and 4I) post EAE induction. Immunoreactivity was detected with HRP anti-rat Ig plus AEC (red) against a hemotoxylin stained nuclear background (blue). Arrows indicate sites of lymphocyte infiltration. Scale bars represent 500mm.

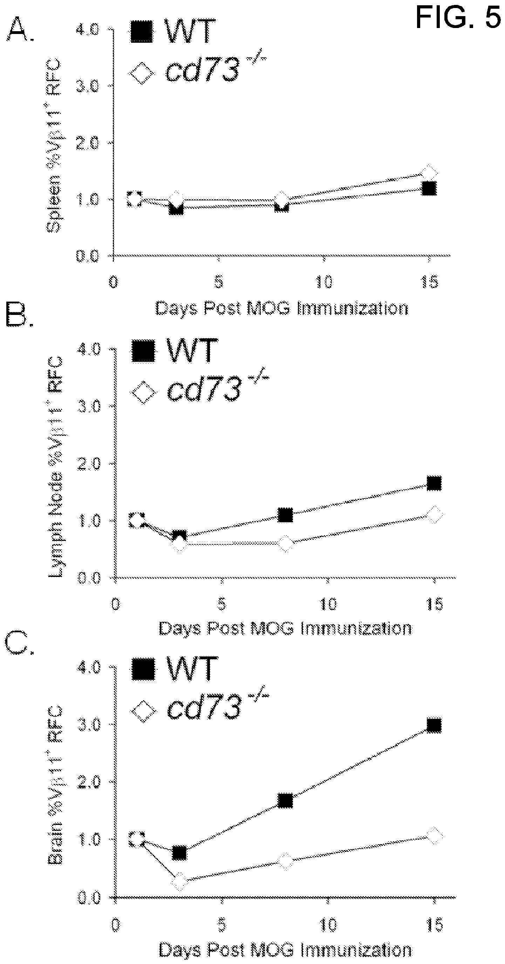

Figure 5A-C show myelin specific T cells do not efficiently enter the brain of *cd73*^{-/-} mice following EAE induction. Vβ11⁺ T cells from MOG₃₅₋₅₅ immunized transgenic 2d2 mice, which express TCRs specific for MOG₃₅₋₅₅, were isolated from the spleen and lymph nodes and adoptively transferred into wild type or *cd73*^{-/-} mice with concomitant EAE induction. At days 1, 3, 8, and 15 post transfer and EAE induction, spleens (Fig. 5A), lymph nodes (Fig. 5B), and brains (Fig. 5C) were removed and cells were harvested. Cells were analyzed for CD45 and Vβ11 expression by flow cytometry. The data represent the relative fold change (RFC) in the percentage of Vβ11⁺ cells in the CD45⁺ population for each organ on each given day. Values were normalized to the percentage of cells found in each organ at 1 day post transfer/EAE induction, with 1.0 equaling the baseline value.

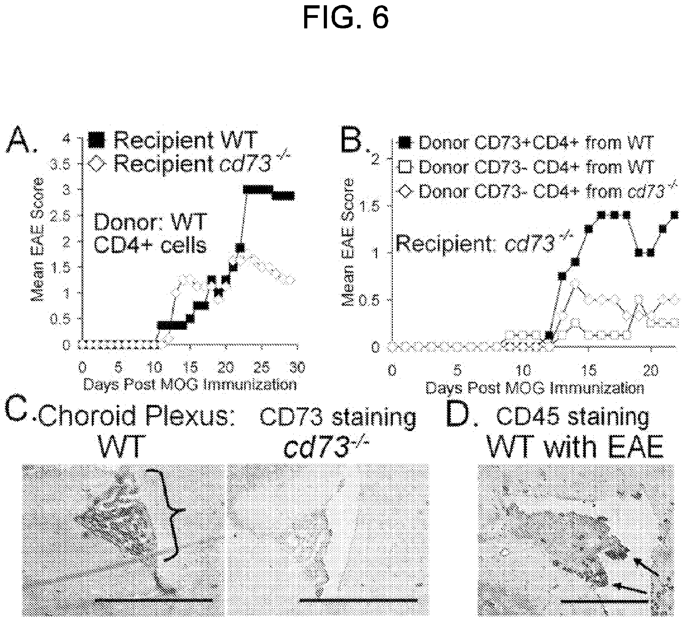

Figure 6A-D show adoptively transferred CD73⁺ T cells from wild type mice can confer EAE susceptibility to *cd73*^{-/-} mice. Figure 6A shows CD4⁺ T cells from the spleen and lymph nodes of MOG immunized wild type mice were enriched and adoptively transferred into wild type (closed squares, n=5) or *cd73*^{-/-} (open diamonds, n=5) mice followed by concomitant EAE induction. Results are shown from one of two independent experiments. Figure 6B shows T cells from the spleen and lymph nodes of previously immunized wild type and *cd73*^{-/-} mice were sorted based on CD4 and CD73 expression and adoptively transferred into *cd73*^{-/-} mice followed by concomitant EAE induction (n=5/each group). Closed squares represent donor cells from wild type mice that express CD73; open squares represent donor cells from wild type mice that lack CD73 expression; open diamonds represent donor cells from *cd73*^{-/-} mice. Figure 6C-D show frozen tissue sections of the CNS choroid plexus from naive wild type (Figure 6C, left) and *cd73*^{-/-} (Figure 6C, right) mice and wild type mice day 12 post-EAE induction (Figure 6D) were stained with a CD73 (Figure 6C) or CD45 (Figure 6D) specific antibody. Immunoreactivity was detected with HRP anti-rat Ig plus AEC (red) against a hemotoxylin stained nuclear background (blue). Brackets indicate CD73 staining. Arrows indicate CD45 lymphocyte staining. Scale bars represent 500µm.

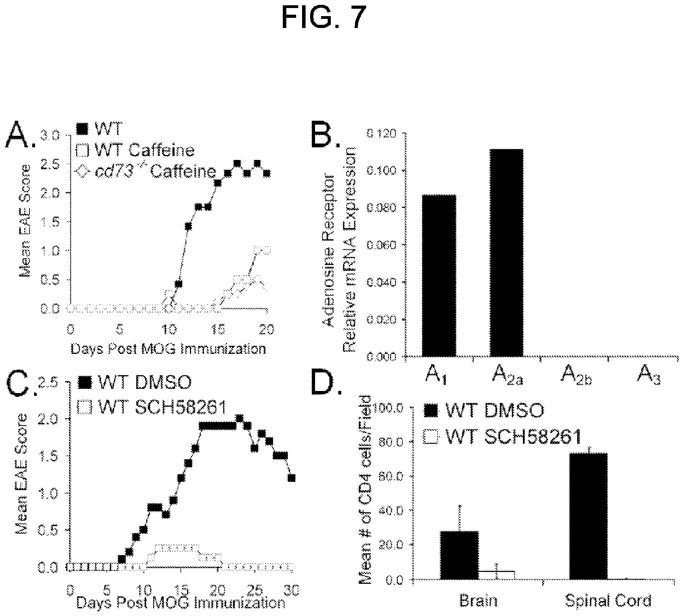

Figure 7A-D show adenosine receptor blockade protects mice from EAE development. Figure 7A shows mean EAE scores where EAE was induced, disease activity was monitored daily, and the mean EAE score was calculated in wild type (squares) and *cd73*^{-/-} (diamonds) mice given either drinking water (closed shape) alone or drinking water supplemented with 0.6g/ml of the broad spectrum adenosine receptor antagonist caffeine (open shape). Results are from one experiment (n=5 mice per group). Figure 7B shows adenosine receptor mRNA expression levels relative to the GAPDH housekeeping gene in the Z310 murine choroid plexus cell line. Samples were run in triplicate; error bars represent the standard error of the mean. Figure 7C shows results after mice were treated with the A₂ₐ adenosine receptor antagonist SCH58261 at 2mg/kg (1mg/kg s.c. and 1mg/kg i.p.) in 45% DMSO (closed squares, n=4 mice/group) or 45% DMSO alone (open squares, n=5 mice/group) 1 day prior to and daily up to day 30 following EAE induction. These results are representative of two experiments. Figure 7D shows the mean number of CD4⁺ infiltrating lymphocytes in the brain and spinal cord quantified per field in frozen tissue sections from day 15 post-EAE induction in SCH58261- and DMSO-treated mice are shown. Eight anatomically similar fields per brain and 4 fields per spinal cord per mouse were analyzed at 10x magnification (n=4 mice). Error bars represent the standard error of the mean.

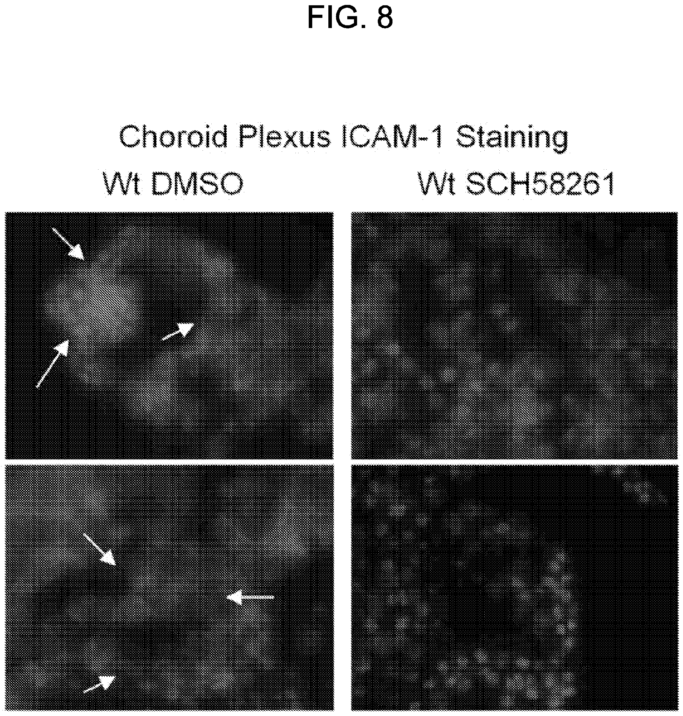

Figure 8 shows the A₂ₐ adenosine receptor antagonist SCH58261 prevents ICAM-1 upregulation on the choroid plexus following EAE induction. Mice were treated with the A₂ₐ adenosine receptor antagonist SCH58261 2mg/kg (1mg/kg given *s.c.* and 1mg/kg given *i.p.*) in DMSO (n=4 mice/group) or DMSO alone (n=5 mice/group) 1 day prior to and daily up to day 30 following EAE induction. These results are from one experiment. Frozen tissue sections from day 15 post-EAE induction in SCH58261 and DMSO treated mice were examined for ICAM-1 expression at the choroid plexus. WT treated DMSO (left) or SCH58261 (right) and stained for ICAM-1 (red staining, white arrows) and DAPI (blue, nuclei) at 40x magnification. Images are from 4 separate mice.

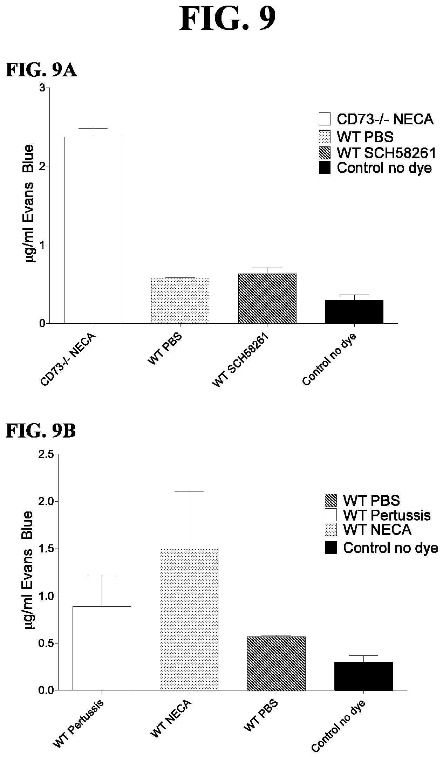

Figure 9A-B demonstrate that CD73^{-/-} mice, which lack extracellular adenosine and thus cannot adequately signal through adenosine receptors, were treated with NECA, resulting in an almost five fold increase in dye migration vs. the PBS control (Fig. 9A). WT mice treated with NECA also show an increase over control mice (Fig. 9B). Pertussis was used as a positive control, as it is known to induce blood brain barrier leakiness in the mouse EAE model.

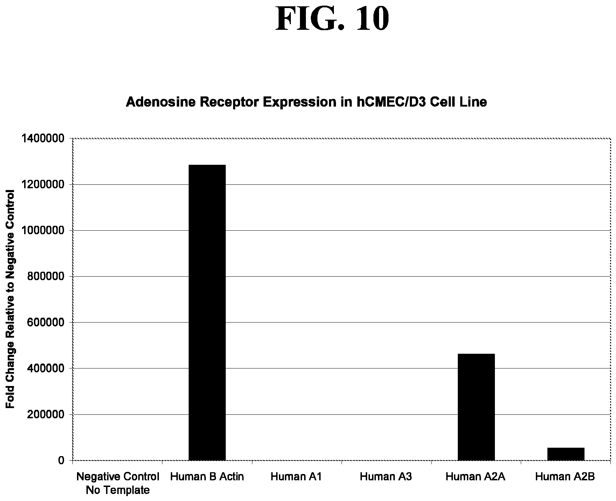

Figure 10 shows adenosine receptor expression on the human endothelial cell line hCMEC/D3.

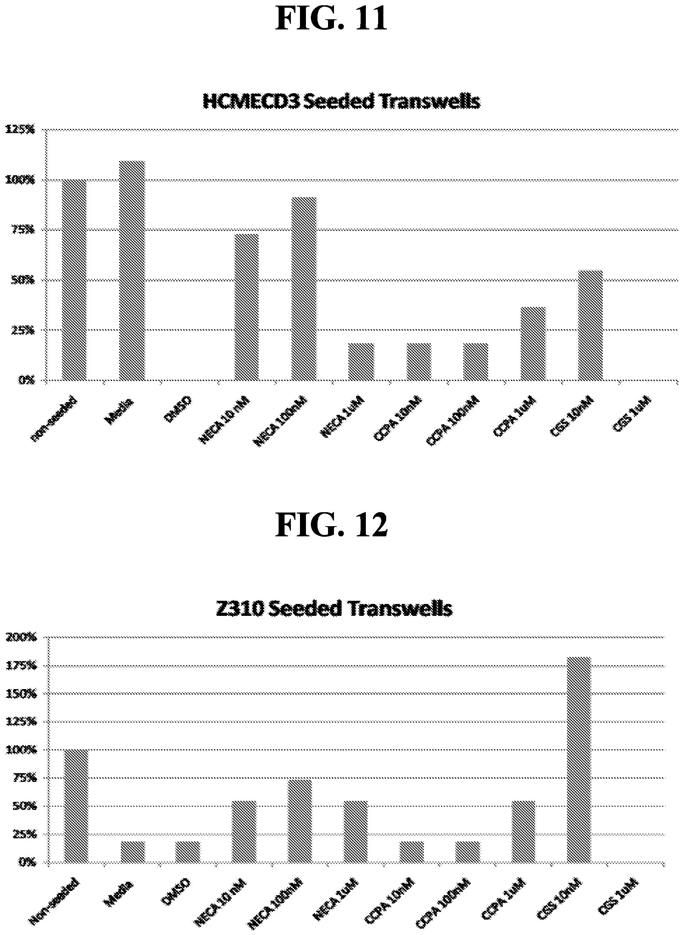

Figure 11 shows results after hCMEC/D3 cells were seeded onto transwell membranes and allowed to grow to confluencey; 2x10⁶ Jurkat cells were added to the upper chamber with or without NECA (general adenosine receptor [AR] agonist), CCPA (A1AR agonist), CGS 21680 (A2AAR agonist), or DMSO vehicle; and migrated cells were counted after 24 hours.
Figure 12 shows results after transwell membranes were seeded with Z310 cells and allowed to grow to confluencey; 2x10⁶ Jurkat cells were added to the upper chamber with or with out NECA (n=1, general AR agonist), CCPA (n=1, A1AR agonist), CGS 21680 (n=1, A2AAR agonist), or DMSO vehicle(n=1); and migrated cells were counted after 24 hours.

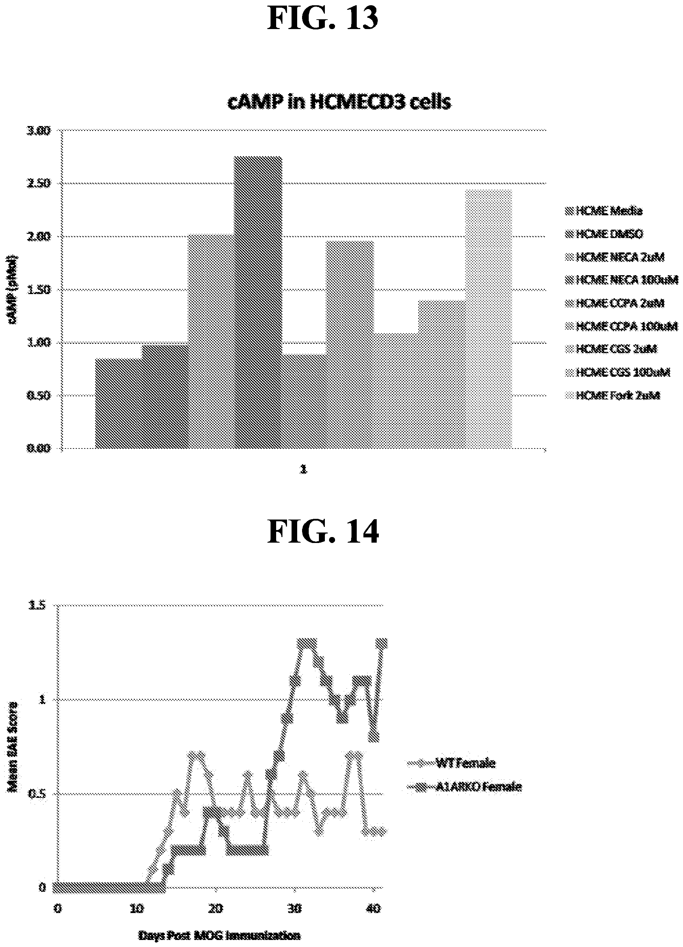

Figure 13 shows results after hCMEC/D3 cells were grown to confluencey on 24 well plates; cells were treated with or without various concentrations of NECA (general AR agonist), CCPA (A1AR agonist), CGS 21680 (A2AAR agonist), DMSO vehicle, or Forksolin (induces cAMP); lysis buffer was added after 15 minutes and the cells were frozen at -80C to stop the reaction; and cAMP levels were assayed using a cAMP Screen kit (Applied Biosystems, Foster City, CA).
Figure 14 shows results of female A1 adenosine receptor knockout (A1ARKO, n=5) and wild type (WT, n=5) mice that were immunized with CFA/MOG₃₅₋₅₅ + PTX on 12-2-08 and scored daily for 41 days.

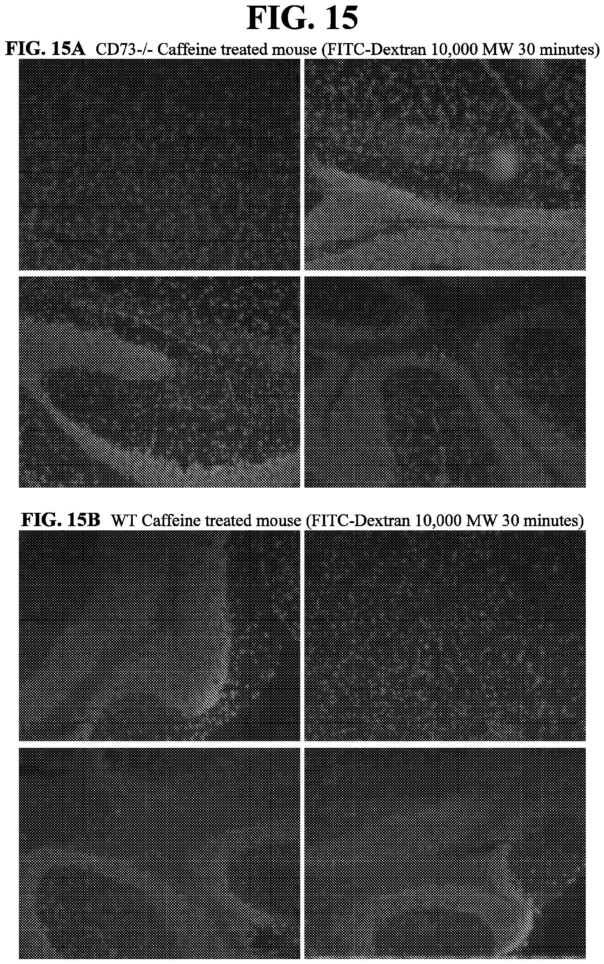

Figure 15A-B show brains of wild type mice fed caffeine and brains from CD73^{-/-} mice fed caffeine, as measured by FITC-Dextran extravasation through the brain endothelium.

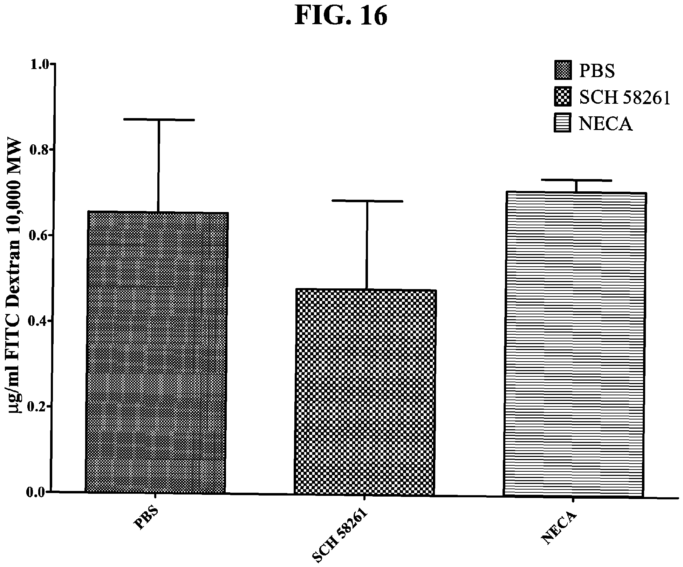

Figure 16 shows results in graph form of FITC-Dextran extravasation across the blood brain barrier of wild type mice treated with adenosine receptor agonist, NECA, while SCH58261, the adenosine receptor antagonist inhibit FITC-Dextran extravasation.

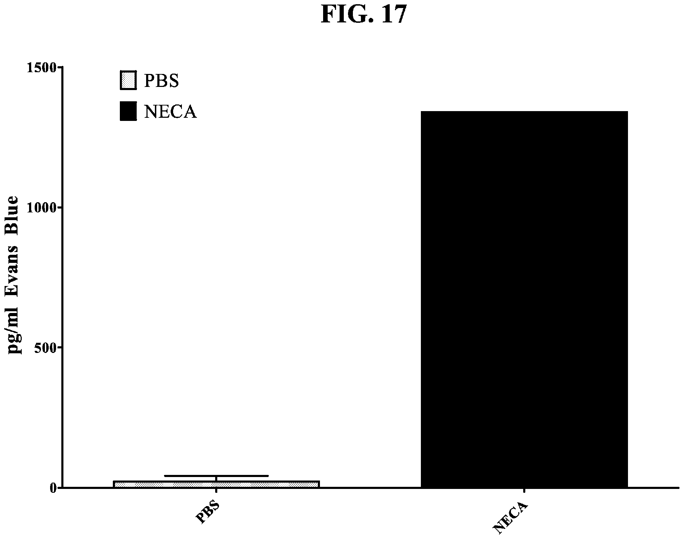

Figure 17 shows results of Evans Blue dye extravasation across the blood brain barrier, as measured by a BioTex spectrophotometer at 620nm, after mice were treated with adenosine receptor agonist NECA.

### DETAILED DESCRIPTION OF THE INVENTION

Provided with the invention is a method of increasing blood brain barrier permeability in a subject. This method involves selecting a subject who would benefit from increased blood brain barrier permeability and subjecting the selected subject to a treatment. That treatment increases adenosine level and/or bioavailability, modulates adenosine receptors, and/or increases CD73 level and/or activity under conditions effective to increase blood brain barrier permeability in the subject.
The invention provides an A2A adenosine receptor agonist and/or an A1 adenosine receptor antagonist, for use in increasing blood brain barrier permeability in a subject, wherein the subject is one who would benefit from increased blood brain barrier permeability and wherein the subject is not subjected to a treatment which increases adenosine level.

Adenosine is a cellular signal of metabolic distress being produced in hypoxic, ischaemic, or inflammatory conditions. Its primary undertaking is to reduce tissue injury and promote repair by different receptor-mediated mechanisms, including the increase of oxygen supply/demand ratio, preconditioning, anti-inflammatory effects and stimulation of angiogenesis (Jacobson et al., "Adenosine Receptors as Therapeutic Targets," Nat. Rev. Drug Discov. 5:247-264(2006)). The biological effects of adenosine are ultimately dictated by the different pattern of receptor distribution and/or affinity of the four known adenosine receptor ("AR") subtypes in specific cell types.

CD73 (ecto-5'-nucleotidase) is a 70-kD glycosyl- phosphatidyl-inositol-anchored cell surface molecule with ecto-enzymatic activity. It is abundantly expressed on many cell types including subsets of lymphocytes (Yamashita et al., "CD73 Expression and Fyn-Dependent Signaling on Murine Lymphocytes," Eur. J. Immunol. 28:2981-2990 (1998)), endothelial cells (Yamashita et al., "CD73 Expression and Fyn-Dependent Signaling on Murine Lymphocytes," Eur. J. Immunol. 28:2981-2990 (1998)), and epithelial cells (Strohmeier et al., "Surface Expression, Polarization, and Functional Significance of CD73 in Human Intestinal Epithelia," J. Clin. Invest. 99:2588-2601 (1997)). It is part of the purine salvage pathway by degrading nucleoside-5'-monophosphates (AMP and IMP) into nucleotides like adenosine and inosine.

Suitable methods of CD73 control disclosed herein include administering a recombinant CD73 protein, or by a cytokine or another factor capable of inducing endothelial CD73 expression or by a combination of both therapies as described in U.S. Patent Application Publication No. 2006/0198821 A1 to Jalkanen. More specifically, suitable agents disclosed herein include cytokines or other factors that directly or indirectly upregulate transcription of the CD73 gene. A suitable cytokine is typically an interferon or an interleukin, but also other agents may be used. In case the cytokine is an interferon, the interferon may be alpha-, beta-, gamma-, omega-, or any other interferon and it can be any subtype of the aforementioned interferons. It is believed that particularly alpha- and beta-interferons are suitable for such a use. Any interleukin capable of inducing endothelial CD73 expression is also suitable for use in this method. Examples of such interleukins include IL-4, IL-10, IL-13 and IL-20.

In one disclosure, the administration of recombinant CD73 protein or a cytokine or both is combined with an administration of adenosine monophosphate ("AMP") in order to safeguard the source for adenosine to be produced as a result of the elevated CD73 level, obtained by elevated expression or by direct administering of the recombinant CD73 protein.

Administration of recombinant CD73 protein or a cytokine or both can be with an administration of an adenylate kinase inhibitor, which prevents AMP from conversion into adenosine diphosphate ("ADP") or adenosine triphosphate ("ATP").

Alternatively, the administration of recombinant CD73 protein or a cytokine or both may be combined with the administration of an adenosine deaminase inhibitor which prevents the decomposition of adenosine. This could also further be combined with administration of AMP and optionally also an adenylate kinase inhibitor which prevents AMP from conversion into adenosine diphosphate (ADP) or adenosine triphosphate (ATP).

Extracellular adenosine (which may be generated by CD73, as described above) regulates the entry of immune cells into the central nervous system. Accordingly, the BBB permeability is mediated by the local adenosine concentration along with the activity of adenosine receptors: the A1 receptor is activated at low adenosine concentration (high affinity) and the A2a is activated at high adenosine concentrations (low affinity). Thus, increasing adenosine availability will increase the permeability of the BBB. Inversely, decreasing the availability of adenosine will decrease the permeability of the BBB. In addition, increasing the CD73 level or activity will produce additional local adenosine, as described in detail above, thereby increasing the permeability of the BBB.

Adenosine, a purine nucleoside product of the CD73 enzyme activity, binds to specific receptors on the cell surface. Adenosine is reported to have a role in many physiological and pathological events. Adenosine is found in all living cells and can be released under appropriate conditions, such as ischemia or 20 anoxia, where it can then act upon adenosine receptors to produce a variety of physiological effects.

Adenosine receptors are now known to be integral membrane proteins which bind extracellular adenosine, thereby initiating a transmembrane signal via specific guanine nucleotide binding proteins known as G-proteins to modulate a variety of second messenger systems, including adenylyl cyclase, potassium channels, calcium channels and phospholipase C. See Stiles, "Adenosine Receptors and Beyond: Molecular Mechanisms of Physiological Regulation," Clin. Res. 38(1):10-18 (1990); Stiles, "Adenosine Receptors," J. Biol Chem. 267: 6451-6454 (1992).

Activating the A2A adenosine receptor will increase permeability of the BBB. Suitable adenosine receptor A2A activators are A2A agonists, which are well known in the art (Press et al., "Therapeutic Potential of Adenosine Receptor Antagonists and Agonists," Expert Opin. Ther. Patents 17(8):1-16(2007)). Other A2A adenosine receptor agonists include those described in U.S. Patent No. 6,232,297 and in U.S. Published Patent Application No. 2003/0186926 A1 to Lindin et al., 2005/0054605 A1 to Zablocki et al., and U.S. Published Patent Application Nos. 2006/0040888 A1, 2006/0040889 A1, 2006/0100169 A1, and 2008/0064653 A1 to Li et al.. Such compounds may be synthesized as described in: U.S. Patent Nos. 5,140,015 and 5,278,150 to Olsson et al.; U.S. Patent No. 5,593,975 to Cristalli; U.S. Patent No. 4,956,345 Miyasaka et al.; Hutchinson et al., "CGS 21680C, an A2 Selective Adenosine Receptor Agonist with Preferential Hypotensive Activity," J. Pharmacol. Exp. Ther., 251: 47-55 (1989); Olsson et al, "N6-Substituted N-alkyladenosine-5'-uronamides: Bifunctional Ligands Having Recognition Groups for A1 and A2 Adenosine Receptors," J. Med. Chem., 29: 1683-1689 (1986); Bridges et al., "N6-[2-(3,5-dimethoxyphenyl)-2-(2-methylphenyl)ethyl]adenosine and its Uronamide Derivatives: Novel Adenosine Agonists With Both High Affinity and High Selectivity for the Adenosine A2 Receptor," J. Med. Chem. 31: 1282 (1988); Hutchinson et al., J. Med. Chem., 33:1919 (1990); Ukeeda et al., "2-Alkoxyadenosines: Potent and Selective Agonists at the Coronary Artery A2 Adenosine Receptor," J. Med. Chem. 34: 1334 (1991); Francis et al., "Highly Selective Adenosine A2 Receptor Agonists in a Series of N-alkylated 2-aminoadenosines," J. Med. Chem. 34: 2570-2579 (1991); Yoneyama et al, "Vasodepressor Mechanisms of 2-(1-octynyl)-adenosine (YT-146), a Selective Adenosine A2 Receptor Agonist, Involve the Opening of Glibenclamide-sensitive K+ Channels," Eur. J. Pharmacol. 213(2):199-204 (1992); Peet et al., "Conformationally Restrained, Chiral (phenylisopropyl)amino-substituted pyrazolo[3,4-d]pyrimidines and Purines with Selectivity for Adenosine A1 and A2 Receptors," J. Med. Chem., 35: 3263-3269 (1992); and Cristalli et al.,"2-Alkynyl Derivatives of Adenosine and Adenosine-5'-N-ethyluronamide as Selective Agonists at A2 Adenosine Receptors, " J. Med. Chem. 35(13): 2363-2368 (1992),. These adenosine A2A receptor agonists are intended to be illustrative and not limiting.

Blocking or deactivation of the A1 adenosine receptor will also increase permeability of the BBB. Suitable adenosine receptor A1 deactivators are adenosine receptor A1 antagonists, which are well known in the art (Press et al., "Therapeutic Potential of Adenosine Receptor Antagonists and Agonists," Expect Opin. Ther. Patents 17(8):1-16(2007)). Suitable adenosine receptor A1 antagonists include, but are not limited to, those described in U.S. Patent Application Publication No. 2008/0027082 A1 to Hocher et al., U.S. Patent Nos. 5,446,046 and 5,668,139 to Belardinelli et al., U.S. Patent No. 6,117,998 to Neely, and U.S. Patent No. 7,247,639 to Wilson et al..

In increasing BBB permeability, the selected subject can have a central nervous system ("CNS") disorder.

Disorders of the CNS (which encompass psychiatric/behavioral diseases or disorders) may include, but are not limited to, acquired epileptiform aphasia, acute disseminated encephalomyelitis, adrenoleukodystrophy, agenesis of the corpus callosum, agnosia, aicardi syndrome, Alexander disease, Alpers' disease, alternating hemiplegia, Alzheimer's disease, amyotrophic lateral sclerosis, anencephaly, Angelman syndrome, angiomatosis, anoxia, aphasia, apraxia, arachnoid cysts, arachnoiditis, Arnold-chiari malformation, arteriovenous malformation, Asperger's syndrome, ataxia telangiectasia, attention deficit hyperactivity disorder, autism, auditory processing disorder, autonomic dysfunction, back pain, Batten disease, Behcet's disease, Bell's palsy, benign essential blepharospasm, benign focal amyotrophy, benign intracranial hypertension, bilateral frontoparietal polymicrogyria, binswanger's disease, blepharospasm, Bloch-sulzberger syndrome, brachial plexus injury, brain abscess, brain damage, brain injury, brain tumor, spinal tumor, Brown-séquard syndrome, canavan disease, carpal tunnel syndrome (cts), causalgia, central pain syndrome, central pontine myelinolysis, centronuclear myopathy, cephalic disorder, cerebral aneurysm, cerebral arteriosclerosis, cerebral atrophy, cerebral gigantism, cerebral palsy, charcot-marie-tooth disease, chiari malformation, chorea, chronic inflammatory demyelinating polyneuropathy ("CIDP"), chronic pain, chronic regional pain syndrome, Coffin lowry syndrome, coma (including persistent vegetative state), congenital facial diplegia, corticobasal degeneration, cranial arteritis, craniosynostosis, Creutzfeldt-jakob disease, cumulative trauma disorders, Cushing's syndrome, cytomegalic inclusion body disease ("CIBD"), cytomegalovirus infection, dandy-walker syndrome, Dawson disease, de morsier's syndrome, Dejerine-klumpke palsy, Dejerine-sottas disease, delayed sleep phase syndrome, dementia, dermatomyositis, developmental dyspraxia, diabetic neuropathy, diffuse sclerosis, dysautonomia, dyscalculia, dysgraphia, dyslexia, dystonia, early infantile epileptic encephalopathy, empty sella syndrome, encephalitis, encephalocele, encephalotrigeminal angiomatosis, encopresis, epilepsy, Erb's palsy, erythromelalgia, essential tremor, Fabry's disease, Fahr's syndrome, fainting, familial spastic paralysis, febrile seizures, fisher syndrome, Friedreich's ataxia, Gaucher's disease, Gerstmann's syndrome, giant cell arteritis, giant cell inclusion disease, globoid cell leukodystrophy, gray matter heterotopia, Guillain-barré syndrome, htlv-1 associated myelopathy, Hallervorden-spatz disease, head injury, headache, hemifacial spasm, hereditary spastic paraplegia, heredopathia atactica polyneuritiformis, herpes zoster oticus, herpes zoster, hirayama syndrome, holoprosencephaly, Huntington's disease, hydranencephaly, hydrocephalus, hypercortisolism, hypoxia, immune-mediated encephalomyelitis, inclusion body myositis, incontinentia pigmenti, infantile phytanic acid storage disease, infantile refsum disease, infantile spasms, inflammatory myopathy, intracranial cyst, intracranial hypertension, Joubert syndrome, Kearns-sayre syndrome, Kennedy disease, kinsbourne syndrome, Klippel feil syndrome, Krabbe disease, Kugelberg-welander disease, kuru, lafora disease, Lambert-eaton myasthenic syndrome, Landau-kleffner syndrome, lateral medullary (Wallenberg) syndrome, learning disabilities, leigh's disease, Lennox-gastaut syndrome, Lesch-nyhan syndrome, leukodystrophy, lewy body dementia, lissencephaly, locked-in syndrome, Lou Gehrig's disease, lumbar disc disease, lyme disease - neurological sequelae, machado-joseph disease (spinocerebellar ataxia type 3), macrencephaly, megalencephaly, Melkersson-rosenthal syndrome, Ménière's disease, meningitis, Menkes disease, metachromatic leukodystrophy, microcephaly, migraine, Miller Fisher syndrome, mini-strokes, mitochondrial myopathies, mobius syndrome, monomelic amyotrophy, motor neurone disease, motor skills disorder, moyamoya disease, mucopolysaccharidoses, multi-infarct dementia, multifocal motor neuropathy, multiple sclerosis, multiple system atrophy with postural hypotension, muscular dystrophy, myalgic encephalomyelitis, myasthenia gravis, myelinoclastic diffuse sclerosis, myoclonic encephalopathy of infants, myoclonus, myopathy, myotubular myopathy, myotonia congenita, narcolepsy, neurofibromatosis, neuroleptic malignant syndrome, neurological manifestations of aids, neurological sequelae of lupus, neuromyotonia, neuronal ceroid lipofuscinosis, neuronal migration disorders, niemann-pick disease, non 24-hour sleep-wake syndrome, nonverbal learning disorder, O'sullivan-mcleod syndrome, occipital neuralgia, occult spinal dysraphism sequence, ohtahara syndrome, olivopontocerebellar atrophy, opsoclonus myoclonus syndrome, optic neuritis, orthostatic hypotension, overuse syndrome, palinopsia, paresthesia, Parkinson's disease, paramyotonia congenita, paraneoplastic diseases, paroxysmal attacks, parry-romberg syndrome (also known as rombergs syndrome), pelizaeus-merzbacher disease, periodic paralyses, peripheral neuropathy, persistent vegetative state, pervasive developmental disorders, photic sneeze reflex, phytanic acid storage disease, pick's disease, pinched nerve, pituitary tumors, pmg, polio, polymicrogyria, polymyositis, porencephaly, post-polio syndrome, postherpetic neuralgia ("PHN"), postinfectious encephalomyelitis, postural hypotension, Prader-willi syndrome, primary lateral sclerosis, prion diseases, progressive hemifacial atrophy (also known as Romberg's syndrome), progressive multifocal leukoencephalopathy, progressive sclerosing poliodystrophy, progressive supranuclear palsy, pseudotumor cerebri, ramsay-hunt syndrome (type I and type II), Rasmussen's encephalitis, reflex sympathetic dystrophy syndrome, refsum disease, repetitive motion disorders, repetitive stress injury, restless legs syndrome, retrovirus-associated myelopathy, rett syndrome, Reye's syndrome, Romberg's syndrome, rabies, Saint Vitus' dance, Sandhoff disease, schizophrenia, Schilder's disease, schizencephaly, sensory integration dysfunction, septo-optic dysplasia, shaken baby syndrome, shingles, Shy-drager syndrome, Sjögren's syndrome, sleep apnea, sleeping sickness, snatiation, Sotos syndrome, spasticity, spina bifida, spinal cord injury, spinal cord tumors, spinal muscular atrophy, spinal stenosis, Steele-richardson-olszewski syndrome, see progressive supranuclear palsy, spinocerebellar ataxia, stiff-person syndrome, stroke, Sturge-weber syndrome, subacute sclerosing panencephalitis, subcortical arteriosclerotic encephalopathy, superficial siderosis, sydenham's chorea, syncope, synesthesia, syringomyelia, tardive dyskinesia, Tay-sachs disease, temporal arteritis, tetanus, tethered spinal cord syndrome, Thomsen disease, thoracic outlet syndrome, tic douloureux, Todd's paralysis, Tourette syndrome, transient ischemic attack, transmissible spongiform encephalopathies, transverse myelitis, traumatic brain injury, tremor, trigeminal neuralgia, tropical spastic paraparesis, trypanosomiasis, tuberous sclerosis, vasculitis including temporal arteritis, Von Hippel-lindau disease ("VHL"), Viliuisk encephalomyelitis ("VE"), Wallenberg's syndrome, Werdnig-hoffman disease, west syndrome, whiplash, Williams syndrome, Wilson's disease, and Zellweger syndrome. It is thus appreciated that all CNS-related states and disorders could be treated through the BBB route of drug delivery.

The compounds of the present invention can be administered orally, parenterally, for example, subcutaneously, intravenously, intramuscularly, intraperitoneally, by intranasal instillation, or by application to mucous membranes, such as, that of the nose, throat, and bronchial tubes. They may be administered alone or with suitable pharmaceutical carriers, and can be in solid or liquid form such as, tablets, capsules, powders, solutions, suspensions, or emulsions.

The active compounds of the present invention may be orally administered, for example, with an inert diluent, or with an assimilable edible carrier, or they may be enclosed in hard or soft shell capsules, or they may be compressed into tablets, or they may be incorporated directly with the food of the diet. For oral therapeutic administration, these active compounds may be incorporated with excipients and used in the form of tablets, capsules, elixirs, suspensions, syrups, and the like. Such compositions and preparations should contain at least 0.1 % of active compound. The percentage of the compound in these compositions may, of course, be varied and may conveniently be between about 2% to about 60% of the weight of the unit. The amount of active compound in such therapeutically useful compositions is such that a suitable dosage will be obtained. Preferred compositions according to the present invention are prepared so that an oral dosage unit contains between about 1 and 250 mg of active compound.

The tablets, capsules, and the like may also contain a binder such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin. When the dosage unit form is a capsule, it may contain, in addition to materials of the above type, a liquid carrier, such as a fatty oil.

Various other materials may be present as coatings or to modify the physical form of the dosage unit. For instance, tablets may be coated with shellac, sugar, or both. A syrup may contain, in addition to active ingredient, sucrose as a sweetening agent, methyl and propylparabens as preservatives, a dye, and flavoring such as cherry or orange flavor.

These active compounds may also be administered parenterally. Solutions or suspensions of these active compounds can be prepared in water suitably mixed with a surfactant, such as hydroxypropylcellulose. Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils.

The compounds of the present invention may also be administered directly to the airways in the form of an aerosol. For use as aerosols, the compounds of the present invention in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

Also provided is a method of decreasing blood brain barrier permeability in a subject. This method involves selecting a subject who would benefit from decreased blood brain barrier permeability and subjecting the selected subject to a treatment. That treatment decreases adenosine level and/or bioavailability, modulates adenosine receptors, and/or decreases CD73 level and/or activity under conditions effective to decrease blood brain barrier permeability in the subject.
The invention provides an A2A adenosine receptor antagonist for use in decreasing blood brain barrier permeability in a subject, wherein the subject is one who would benefit from decreased blood brain barrier permeability.

This aspect of the present invention can be carried out using the pharmaceutical formulations and methods of administration described above.

As described in detail above, permeability of the BBB is controlled by local adenosine levels, CD73, and adenosine receptor activity. Altering the activity of adenosine receptors can be accomplished by providing adenosine receptor antagonists and/or agonists, which are well known in the art (Press et al., "Therapeutic Potential of Adenosine Receptor Antagonists and Agonists" Expert Opin. Ther. Patents 17(8):1-16 (2007)).

Altering adenosine receptor activity in a subject to decrease blood barrier permeability can be accomplished by, but not limited to, administering to the subject an A2A adenosine receptor antagonist and/or an A1 adenosine receptor agonist.

A number of adenosine A2A receptor antagonists are known to those of skill in the art and can be used individually or in conjunction in the methods described herein. Such antagonists include, but are not limited to (-)-R,S)-mefloquine (the active enantiomer of the racemic mixture marketed as Mefloquine™), 3,7-Dimethyl-1-propargylxanthine (DMPX), 3-(3-hydroxypropyl)-7-methyl-8- (m-methoxystyryl)-1-propargylxanthine (MX2), 3-(3-hy- droxypropyl)-8-(3-methoxystyryl)-7-methyl-1-propargylxanthin phosphate disodium salt (MSX-3, a phosphate prodrug of MSX-2), 7-methyl-8-styrylxanthine derivatives, SCH 58261, KW-6002, aminofuryltriazolo-tri-azinylaminoethylphenol (ZM 241385), and 8-chlorostyryl- caffeine, KF17837, VR2006, istradefylline, the VERNALIS drugs such as VER 6489, VER 6623, VER 6947, VER 7130, VER 7146, VER 7448, VER 7835, VER 8177VER-11135, VER-6409, VER 6440, VER 6489, VER 6623, VER 6947, VER 7130, VER 7146, VER 7448, VER 7835, VER 8177, pyrazolo [4,3-e]1,2,4-triazolo[1,5-c]pyrimidines, and 5-amino-imidazolo-[4,3-e]-1,2,4-triazolo[1,5-c]pyrimidines, and the like (U.S. Patent Application Publication No. 2006/0128708 to Li et al.), pyrazolo[4,3-e]-[1,2,4]-triazolo[1,5-c]pyrimidines (See e.g., WO 01/92264 to Kase et al.), 2,7-disubstituted-5-amino- [1,2,4]triazolo[1,5-c]pyrimidines (See e.g. WO 03/048163 to Kase et al.), 2,5-disubstituted- 7-amino-[1,2,4]triazolo[1,5-a][1,3,5]triazines (See e.g., Vu et al., "Piperazine Derivatives of [1,2,4]Triazolo[1,5-a][1,3,5]triazine as Potent and Selective Adenosine A2a Receptor Antagonists," J. Med. Chem. 47(17):4291-4299 (2004)), 9-substituted-2- (substituted-ethyn-1-yl)-adenines (See e.g., U.S. Patent No. 7,217,702 to Beauglehole et al.), 7-methyl-8-styrylxanthine derivatives, pyrazolo [4,3-e)1,2,4-triazolo[1,5-c]pyrimidines, and 5-amino-imidazolo-[4,3-e]-1,2,4-triazolo[1,5-c]pyrimi dines (See e.g., U.S. Patent Application Publication No. 2006/0128708 to Li et al.). These adenosine A2A receptor antagonists are intended to be illustrative and not limiting.

Suitable A1 adenosine receptor agonists, disclosed but not claimed, include those described in U.S. Patent Application Publication No. 2005/0054605 A1 to Zablocki et al..

In decreasing BBB permeability, the selected subject can have an inflammatory disease. Such inflammatory diseases include those in which mediators of inflammation pass the blood brain barrier. Such inflammatory diseases include, but are not limited to, inflammation caused by bacterial infection, viral infection, or autoimmune disease. More specifically, such diseases include, but are not limited to, meningitis, multiple sclerosis, neuromyelitis optica, human immunodeficiency virus ("HIV")-1 encephalitis, herpes simplex virus ("HSV") encephalitis, Toxoplams gondii encephalitis, and progressive multifocal leukoencephalopathy.

Where BBB permeability is decreased, the selected subject may also have a condition mediated by entry of lymphocytes into the brain. Other conditions treatable in this fashion include encephalitis of the brain, Parkinson's disease, epilepsy, neurological manifestations of HIV-AIDS, neurological sequela of lupus, and Huntington's disease, meningitis, multiple sclerosis, neuromyelitis optica, HSV encephalitis, and progressive multifocal leukoencephalopathy.

Also disclosed is a method of treating a subject for a disorder or condition of the central nervous system. This method involves selecting a subject with the disorder or condition of the central nervous system and providing a therapeutic effective to treat the disorder or condition of the central nervous system. A blood brain barrier permeabilizing agent, where the agent increases adenosine level and/or bioavailability, modulates adenosine receptors, and/or increases CD73 level and/or activity, is provided. The therapeutic and the blood brain barrier permeabilizing agent are administered to the selected subject under conditions effective for the therapeutic to pass across the blood brain barrier and treat the disorder or condition.

This method can be carried out using the pharmaceutical agents and methods of administration described above.

A therapeutic may include any therapeutic useful in the treatment of a disease or condition of the CNS. Such other compounds may be of any class of drug or pharmaceutical agent, including but not limited to antibiotics, anti-parasitic agents, antifungal agents, anti-viral agents and anti-tumor agents. When administered with anti-parasitic, anti-bacterial, anti-fungal, anti-tumor, anti-viral agents, and the like, the blood brain barrier permeabilizing agent compounds may be administered by any method and route of administration suitable to the treatment of the disease, typically as pharmaceutical compositions.

Therapeutic agents can be delivered as a therapeutic or as a prophylactic (e.g., inhibiting or preventing onset of neurodegenerative diseases). A therapeutic causes eradication or amelioration of the underlying disorder being treated. A prophylactic is administered to a patient at risk of developing a disease or to a patient reporting one or more of the physiological symptoms of such a disease, even though a diagnosis may not have yet been made. Alternatively, prophylactic administration may be applied to avoid the onset of the physiological symptoms of the underlying disorder, particularly if the symptom manifests cyclically. In this latter disclosure, the therapy is prophylactic with respect to the associated physiological symptoms instead of the underlying indication. The actual amount effective for a particular application will depend, *inter alia,* on the condition being treated and the route of administration.

The therapeutic can be immunosuppressants, anti-inflammatories, anti-proliferatives, anti-migratory agents, anti-fibrotic agents, proapoptotics, calcium channel blockers, anti-neoplasties, antibodies, anti-thrombotic agents, anti-platelet agents, Ilblllla agents, antiviral agents, anti-cancer agents, chemotherapeutic agents, thrombolytics, vasodilators, antimicrobials or antibiotics, antimitotics, growth factor antagonists, free radical scavengers, biologic agents, radio therapeutic agents, radio-opaque agents, radiolabelled agents, anti-coagulants (e.g., heparin and its derivatives), anti-angiogenesis drugs (e.g., Thalidomide), angiogenesis drugs, PDGF-B and/or EGF inhibitors, anti-inflammatories (e.g., psoriasis drugs), riboflavin, tiazofurin, zafurin, anti-platelet agents (e.g., cyclooxygenase inhibitors (e.g., acetylsalicylic acid)), ADP inhibitors (such as clopidogrel and ticlopdipine), hosphodiesterase III inhibitors (such as cilostazol), lycoprotein II/IIIIa agents (such as abcix- imab ), eptifibatide, and adenosine reuptake inhibitors (such as dipyridmoles, healing and/or promoting agents (e.g., anti-oxidants and nitrogen oxide donors)), antiemetics, antinauseants, tripdiolide, diterpenes, triterpenes, diterpene epoxides, diterpenoid epoxide, triepoxides, or tripterygium wifordii hook F(TWHF), SDZ- RAD, RAD, RAD666, or 40-0-(2-hydroxy)ethyl-rapamycin, derivatives, pharmaceutical salts and combinations thereof.

In one disclosure, the therapeutic capable agent is a bioactive protein or peptide. Examples of such bioactive protein or peptides include a cell modulating peptide, a chemotactic peptide, an anticoagulant peptide, an antithrombotic peptide, an anti-tumor peptide, an anti-infectious peptide, a growth potentiating peptide, and an anti-inflammatory peptide. Examples of proteins include antibodies, enzymes, steroids, growth hormone and growth hormone-releasing hormone, gonadotropin-releasing hormone and its agonist and antagonist analogues, somatostatin and its analogues, gonadotropins, peptide T, thyrocalcitonin, parathyroid hormone, glucagon, vasopressin, oxytocin, angiotensin I and II, bradykinin, kallidin, adrenocorticotropic hormone, thyroid stimulating hormone, insulin, glucagon and the numerous analogues and congeners of the foregoing molecules. In some aspects of the invention, the BBB permeability is modulated by one or more of the claimed agents to deliver an antibiotic, or an anti-infectious therapeutic capable agent. Such anti-infectious agents reduce the activity of or kills a microorganism.

In certain disclosures the therapeutic and the blood brain barrier permeabilizing agent are formulated as a single "compound" formulation. This can be accomplished by any of a number of known methods. For example, the therapeutic and the blood brain barrier permeabilizing agent can be combined in a single pharmaceutically acceptable excipient. In another approach the therapeutic and the blood brain barrier permeabilizing agent can be formulated in separate excipients that are microencapsulated and then combined, or that form separate laminae in a single pill, and so forth.

In one disclosure, the therapeutic and blood brain barrier permeabilizing agent are linked together. In certain disclosures the therapeutic and the blood brain barrier permeabilizing agent are joined directly together or are joined together by a "tether" or "linker" to form a single compound. Without being bound to a particular theory, it is believed that such joined compounds provide improved specificity/selectivity.

A number of chemistries for linking molecules directly or through a linker/tether are well known to those of skill in the art. The specific chemistry employed for attaching the therapeutic(s) and the blood brain barrier permeabilizing agent to form a bifunctional compound depends on the chemical nature of the therapeutic(s) and the "interligand" spacing desired. Various therapeutics and blood brain barrier permeabilizing agents typically contain a variety of functional groups (e.g. carboxylic acid (COOH), free amine (-NEE), and the like), that are available for reaction with a suitable functional group on a linker or on the opposing component (i.e. either the therapeutic or blood brain barrier permeabilizing agent) to bind the components together.

Alternatively, the components can be derivatized to expose or attach additional reactive functional groups. The derivatization may involve attachment of any of a number of linker molecules such as those available from Pierce Chemical Company, Rockford Ill.

A "linker" or "tether", as used herein, is a molecule that is used to join two or more ligands (e.g., therapeutic(s) or blood brain barrier permeabilizing agent) to form a bi-functional or poly-functional compound. The linker is typically chosen to be capable of forming covalent bonds to all of the components comprising the bi-functional or polyfunctional moiety. Suitable linkers are well known to those of skill in the art and include, but are not limited to, straight or branched-chain carbon linkers, heterocyclic carbon linkers, amino acids, nucleic acids, dendrimers, synthetic polymers, peptide linkers, peptide and nucleic acid analogs, carbohydrates, polyethylene glycol and the like. Where one or more of the components are polypeptides, the linker can be joined to the constituent amino acids through their side groups (e.g., through a disulfide linkage to cysteine) or through the alpha carbon amino or carboxyl groups of the terminal amino acids.

In certain disclosures, a bifunctional linker having one functional group reactive with a group on the first therapeutic and another group reactive with a functional group on the blood brain barrier permeabilizing agent can be used to form a bifunctional compound. Alternatively, derivatization may involve chemical treatment of the component(s) (e.g., glycol cleavage of the sugar moiety of a glycoprotein, a carbohydrate, or a nucleic acid, etc.) with periodate to generate free aldehyde groups. The free aldehyde groups can be reacted with free amine or hydrazine groups on a linker to bind the linker to the compound (See, e.g., U.S. Patent No. 4,671,958 to Rodwell et al.). Procedures for generation of free sulfhydryl groups on polypeptide, such as antibodies or antibody fragments, are also known (See U.S. Patent No. 4,659,839 to Nicolotti et al.).

Where the therapeutic and the blood brain barrier permeabilizing agent are both peptides, a bifunctional compound can be chemically synthesized or recombinantly expressed as a fusion protein comprising both components attached directly to each other or attached through a peptide linker.

In certain disclosures, lysine, glutamic acid, and polyethylene glycol (PEG) based linkers of different length are used to couple the components. The chemistry for the conjugation of molecules to PEG is well known to those of skill in the art (see, e.g., Veronese, "Peptide and Protein PEGylation: a Review of Problems and Solutions," Biomaterials 22: 405-417 (2001); Zalipsky et al., "Attachment of Drugs to Polyethylene Glycols," Eur. Plym. J. 19(12):1177-1183 (1983); Olson et al., "Preparation and Characterization of Poly(ethylene glycol)ylated Human Growth Hormone Antagonist," Poly(ethylene glycol) Chemistry and Biological Applications 170-181, Harris & Zalipsky Eds., ACS, Washington, DC (1997); Delgado et al., "The Uses and Properties of PEG-Linked Proteins," Grit. Rev. Therap. Drug Carrier Sys. 9: 249-304(1992); Pedley et al., "The Potential for Enhanced Tumour Localisation by Poly(ethylene glycol) Modification of anti-CEA Antibody," Brit. J. Cancer 70:1126-1130 (1994); Eyre & Farver, Textbook of Clinical Oncology 377-390 (Holleb et al. eds. 1991); Lee et al., "Prolonged Circulating Lives of Single-chain of Fv Proteins Conjugated with Polyethylene Glycol: a Comparison of Conjugation Chemistries and Compounds," Bioconjug. Chem. 10: 973-981(1999); Nucci et al., "The Therapeutic Value of Poly(Ethytene Glycol)-Modified Proteins," Adv. Drug Deliv. Rev.6: 133-151(1991); Francis et al., "Polyethylene Glycol Modification: Relevance of Improved Methodology to Tumour Targeting," J. Drug Targeting 3: 321-340(1996)).

In certain disclosures, conjugation of the therapeutic and the blood brain barrier permeabilizing agent can be achieved by the use of such linking reagents such as glutaraldehyde, EDCI, terephthaloyl chloride, cyanogen bromide, and the like, or by reductive amination. In certain disclsoures, components can be linked via a hydroxy acid linker of the kind disclosed in WO-A- 9317713. PEG linkers can also be utilized for the preparation of various PEG tethered drugs (See, e.g., Lee et al., "Reduction of Azides to Primary Amines in Substrates Bearing Labile Ester Functionality: Synthesis of a PEG-Solubilized, "Y"-Shaped Iminodiacetic Acid Reagent for Preparation of Folate-Tethered Drugs," Organic Lett., 1: 179-181(1999)).

Also provided is a method of screening compounds effective in increasing blood brain barrier permeability. This method involves providing a modified animal with reduced CD73 expression levels, reduced adenosine expression levels, and/or modulated adenosine receptor activity compared to an unmodified animal. One or more candidate compounds are also provided, and the one or more candidate compounds are administered to the modified animal. It is then evaluated whether the one or more candidate compounds increases adenosine level and/or bioavailability, modulates adenosine receptors, and/or increases CD73 level and/or activity. Those candidate compounds which increase adenosine level and/or bioavailability, modulate adenosine receptors, and/or increase CD73 level and/or activity in the modified animal are then identified as being potentially effective in increasing blood brain barrier permeability.

In certain disclosures, the subject method may also be practiced with the use of a transgenic animal. Transgenic animals can be broadly categorized into two types: "knockouts" and "knockins". A "knockout" has an alteration in the target gene via the introduction of transgenic sequences that results in a decrease of function of the target gene, preferably such that target gene expression is insignificant or undetectable. A "knockin" is a transgenic animal having an alteration in a host cell genome that results in an augmented expression of a target gene, e.g., by introduction of an additional copy of the target gene, or by operatively inserting a regulatory sequence that provides for enhanced expression of an endogenous copy of the target gene. The knock-in or knock-out transgenic animals can be heterozygous or homozygous with respect to the target genes. The transgenic animals of this disclosure can broadly be classified as knockouts or knockins which can overexpress or underexpress CD73, adenosine, and/or adenosine receptors.

In certain disclosures, transgenic animals are designed to provide a model system for determining, identifying and/or quantifying BBB permeability modulation. Such determinations can occur at any time during the animal's life span, including before or after BBB permeability disruption or modification. The transgenic model system can also be used for the development of biologically active agents that promote or increase BBB permeability. Furthermore, the model system can be utilized to assay whether a test agent restores the barrier or decreases permeability (e.g., post BBB 'opening' (i.e., increase permeability), such as, BBB opening resulting from trauma or disease). Moreover, cells can be isolated from the transgenic animals for further study or assays conducted in a cell-based or cell culture setting, including *ex vivo* techniques.

In certain disclosures, the animal models encompass any non-human vertebrates that are amenable to procedures yielding a modified BBB permeability condition in the animal's nervous systems. Preferred model organisms include but are not limited to mammals, non-human primates, and rodents. Non-limiting examples of the preferred models are rats, mice, guinea pigs, cats, dogs, rabbits, pigs, chimpanzees, and monkeys. The test animals can be wildtype or transgenic.

Advances in technologies for embryo micromanipulation now permit introduction of heterologous DNA into fertilized mammalian ova as well. For instance, totipotent or pluripotent stem cells can be transformed by microinjection, calcium phosphate mediated precipitation, liposome fusion, retro viral infection or other means. The transformed cells are then introduced into the embryo, and the embryo will then develop into a transgenic animal. In one disclosure, developing embryos are infected with a viral vector containing a desired transgene so that the transgenic animals expressing the transgene can be produced from the infected embryo. In another disclosure, a desired transgene is co-injected into the pronucleus or cytoplasm of the embryo, preferably at the single cell stage, and the embryo is allowed to develop into a mature transgenic animal. These and other variant methods for generating transgenic animals are well established in the art and hence are not detailed herein. See, for example, U.S. Patent Nos. 5,175,385 and 5,175,384.

BBB permeability can be tested by utilizing various indicators known in the art. For example, dyes, tracers, or markers of a molecular weight greater that 180 Da are precluded from passage through an intact BBB. The assay can be conducted in experimental animals, including without limitation mice, rats, dogs, pigs, or monkeys. Suitable indicators include any dye, marker, or tracer known in the art that is utilized to determine, visualize, measure, identify, or quantify blood-brain barrier permeability. Non-limiting examples include, Evans Blue and sodium fluorescein.

A still further disclosure is directed to a pharmaceutical agent. This pharmaceutical agent has a therapeutic effective to treat the disorder or condition of the central nervous system and a blood brain barrier permeabilizing agent, wherein the agent increases adenosine level and/or bioavailability, modulates adenosine receptors, and/or increases CD73 level and/or activity.

The pharmaceutical can be formulated and administered in substantially the same manner as described above.

### EXAMPLES

### Example 1 - Mice

*Cd73*^{-/-} mice have been previously described (Thompson et al., "Crucial Role for Ecto-5'-Nucleotidase (CD73) in Vascular Leakage During Hypoxia," J. Exp. Med. 200:1395-1405 (2004)) and have been backcrossed to C57BL/6 for 14 generations. *Cd73*^{-/-} mice have no overt susceptibility to infection and appear normal based on the size and cellular composition of their lymphoid organs and their T and B cell responses in *in vivo* and *in vitro* assays (Thompson et al., "Crucial Role for Ecto-5'-Nucleotidase (CD73) in Vascular Leakage During Hypoxia," J. Exp. Med. 200:1395-1405 (2004)). C57BL/6 and *tcrα*^{-/-} mice on the C57BL/6 background were purchased from The Jackson Laboratories. Mice were bred and housed under specific pathogen-free conditions at Cornell University or the University of Turku. For adenosine receptor blockade experiments, mice were given drinking water supplemented with 0.6g/L of caffeine (Sigma) or 2mg/kg SCH58261 (1mg/kg *s.c.* and 1mg/kg *i.p.*) in DMSO (45% vol. in PBS) or 45% DMSO alone starting 1 day before EAE induction and continuing throughout the experiment. All procedures performed on mice were approved by the relevant animal review committee.

### Example 2 - EAE Induction and Scoring

EAE was induced by subjecting mice to the myelin oligodendrocyte glycoprotein ("MOG") EAE-inducing regimen as described in Swanborg, "Experimental Autoimmune Encephalomyelitis in Rodents as a Model for Human Demyelinating Disease," Clin. Immunol. Immunopathol. 77:4-13 (1995) and Bynoe et al., "Epicutaneous Immunization with Autoantigenic Peptides Induces T Suppressor Cells that Prevent Experimental Allergic Encephalomyelitis," Immunity 19:317-328 (2003). Briefly, a 1:1 emulsion of MOG₃₅₋₅₅ peptide (3 mg/ml in PBS) (Invitrogen) and complete Freund's adjuvant (CFA, Sigma) was injected subcutaneously (50µl) into each flank. Pertussis toxin (PTX, 20ng) (Biological Laboratories Inc.) was given intravenously (200µl in PBS) at the time of immunization and again 2 days later. Mice were scored daily for EAE based on disease symptom severity; 0=no disease, 0.5 - 1=weak/limp tail, 2=limp tail and partial hind limb paralysis, 3=total hind limb paralysis, 4=both hind limb and fore limb paralysis, 5=death. Mice with a score of 4 were euthanized.

### Example 3 - T Cell Preparations and Adoptive Transfer

Mice were primed with MOG₃₅₋₅₅ peptide in CFA without PTX. After one week, lymphocytes were harvested from spleen and lymph nodes and incubated with ACK buffer (0.15M NH₄Cl, 1 mM KHCO₃, 0.1mM EDTA, pH 7.3) to lyse red blood cells. Cells were incubated with antibodies to CD8 (TIB-105), IA^{b,d,v,p,q,r} (212.A1), FcR (2.4-G2), B220 (TIB-164), NK1.1 (HB191) and then BioMag goat antimouse IgG, IgM, and goat anti-rat IgG (Qiagen). After negative magnetic enrichment, CD4⁺ cells were used either directly or further sorted into specific subpopulations. For sorting, cells were stained with antibodies to CD4 (RM4-5) and CD73 (TY/23), and in some experiments CD25 (PC61), and then isolated utilizing a FACSAria (BD Biosciences). Post-sort purity was routinely >99%. For adoptive transfer, total CD4⁺ or sorted T cells were washed and resuspended in sterile PBS. Recipient mice received ≤ 2.5 x 10⁶ cells *i.v.* in 200µl of sterile PBS.

### Example 4 - Flow Cytometry

Cell suspensions were stained with fluorochrome-conjugated antibodies for CD4 (RM4-5), CD73 (TY/23), or FoxP3 (FJK-16s). Intracellular FoxP3 staining was carried out according to the manufacturer's instructions (eBioscience). Stained cells were acquired on a FACSCalibur (BD Biosciences). Data were analyzed with FlowJo software (Tree Star).

### Example 5 - T cell Cytokine Assay

Sorted T cells from MOG-immunized mice were cultured in the presence of irradiated C57BL/6 splenocytes with 0 or 10µM MOG peptide. Supernatants were collected at 18 hrs and analyzed utilizing the Bio-plex cytokine (Biorad) assay for IL-2, IL-4, IL-5, IL-10, IL-13, IL-17, IL-1β, and TNFα.

### Example 6 - Immunohistochemistry ("IHC")

Anesthetized mice were perfused with PBS, and brains, spleens, and spinal cords were isolated and snap frozen in Tissue Tek-OCT medium. Five µm sections (brains in a sagittal orientation) were affixed to Supefrost/Plus slides (Fisher), fixed in acetone, and stored at -80°C. For immunostaining, slides were thawed and treated with 0.03% H₂O₂ in PBS to block endogenous peroxidase, blocked with Casein (Vector) in normal goat serum (Zymed), and then incubated with anti-CD45 (YW62.3), anti-CD4 (RM4-5), or anti-ICAM-1 (3E2). Slides were incubated with biotinylated goat anti-rat Ig (Jackson ImmunoResearch) and streptavidin-HRP (Zymed) and developed with an AEC (Red) substrate kit (Zymed) and a hematoxylin counterstain. Cover slips were mounted with Fluoromount-G and photographed under light (Zeiss).

### Example 7 - Real Time q-PCR

Using Trizol (Invitrogen), RNA was isolated from the Z310 choroid plexus cell line (Zheng et al., "Establishment and Characterization of an Immortalized Z310 Choroidal Epithelial Cell Line from Murine Choroid Plexus," Brain Res. 958:371-380 (2002)). cDNA was synthesized using a Reverse-iT kit (ABGene). Primers (available upon request) specific for ARs were used to determine gene expression levels and standardized to the GAPDH housekeeping gene levels using a SYBR-Green kit (ABGene) run on an ABI 7500 real time PCR system. Melt curve analyses were performed to measure the specificity for each qPCR product.

### Example 8 - Evaluation of the Role of CD73 in EAE

Due to the immunomodulatory and immunosuppressive properties of adenosine, the role of CD73 in EAE was evaluated. Based on a report of exacerbated EAE in A1 adenosine receptor (AR)-deficient mice (Tsutsui et al., "A1 Adenosine Receptor Upregulation and Activation Attenuates Neuroinflammation and Demyelination in a Model of Multiple Sclerosis," J. Neurosci. 24:1521-1529 (2004)), *cd73*^{-/-} mice that are unable to catalyze the production of extracellular adenosine were expected to experience severe EAE. Surprisingly, *cd73*^{-/-} mice were highly resistant to the induction of EAE. However, CD4⁺ T cells from *cd73*^{-/-} mice do possess the capacity to generate an immune response against CNS antigens and cause severe EAE when adoptively transferred into *cd73*^{+/+} T cell-deficient mice. CD73⁺CD4⁺ T cells from wild type mice also caused disease when transferred into *cd73*^{-/-} recipients, suggesting that CD73 expression, either on lymphocytes or in the CNS, is required for lymphocyte entry into the brain during EAE. Since *cd73*^{+/+} mice were protected from EAE induction by the broad spectrum AR antagonist caffeine and the A₂ₐ AR specific antagonist SCH58261, this data suggests that the extracellular adenosine generated by CD73, and not CD73 itself, regulates the entry of lymphocytes into the CNS during EAE. These results are the first to demonstrate a role for CD73 and adenosine in regulating the development of EAE.

### Example 9 - Cd73^{-/-} Mice are Resistant to EAE Induction.

To determine if CD73 plays a role in controlling inflammation during EAE progression, *cd73*^{*-*/*-*} and wild type (*cd73*^{+/+}) mice were subjected to the myelin oligodendrocyte glycoprotein ("MOG") EAE-inducing regimen (Swanborg, "Experimental Autoimmune Encephalomyelitis in Rodents as a Model for Human Demyelinating Disease," Clin. Immunol. Immunopathol. 77:4-13 (1995); Bynoe et al., "Epicutaneous Immunization with Autoantigenic Peptides Induces T Suppressor Cells that Prevent Experimental Allergic Encephalomyelitis," Immunity 19:317-328 (2003)). Daily monitoring for EAE development revealed that *cd73*^{*-*/*-*} mice consistently displayed dramatically reduced disease severity compared to their wild type counterparts (Fig. 1). By three weeks after disease induction, *cd73*^{*-*/*-*} mice had an average EAE score of only 0.5 (weak tail) compared to 2.0 (limp tail and partial hind limb paralysis) for wild type mice (Fig. 1).

### Example 10 - CD4⁺ T Cells From cd73^{-/-} Mice Respond to MOG Immunization.

It was then asked whether the resistance of *cd73*^{*-*/*-*} mice to EAE induction could be explained by either an enhanced ability of *cd73*^{*-*/*-*} lymphocytes to suppress an immune response or an inability of these lymphocytes to respond to MOG stimulation. Naturally occurring CD4⁺CD25⁺FoxP3⁺ T cells, or Tregs, can regulate actively-induced EAE (Kohm et al., "Cutting Edge: CD4+CD25+ Regulatory T Cells Suppress Antigen-Specific Autoreactive Immune Responses and Central Nervous System Inflammation During Active Experimental Autoimmune Encephalomyelitis," J. Immunol. 169:4712-4716 (2002)). As Tregs have recently been shown to express CD73 and some reports suggest that the enzymatic activity of CD73 is needed for Treg function (Kobie et al., "T Regulatory and Primed Uncommitted CD4 T Cells Express CD73, Which Suppresses Effector CD4 T Cells by Converting 5'-Adenosine Monophosphate to Adenosine," J. Immunol. 177:6780-6786); Deaglio et al., "Adenosine Generation Catalyzed by CD39 and CD73 Expressed on Regulatory T Cells Mediates Immune Suppression," J. Exp. Med. 204:1257-1265 (2007)), it was asked whether the number and suppressive activity of Tregs were normal in *cd73*^{*-*/*-*} mice. As shown in Fig. 2A, there were no significant differences in the frequencies of CD4⁺FoxP3⁺ Tregs in wild type and *cd73*^{*-*/*-*} mice, either before or after EAE induction. Similarly, the percentage of CD4⁺ T cells that expressed CD73 changed only modestly after EAE induction in wild type mice (Fig. 2B). Additionally, no significant difference was observed in the suppressive capacity of wild type and *cd73*^{*-*/*-*} Tregs to inhibit MOG antigen-specific CD4⁺ effector T cell proliferation. To determine whether *cd73*^{*-*/*-*} T cells can respond when stimulated with MOG peptide, the capacity of these cells to proliferate and produce cytokines was assessed. CD4⁺ T cells from MOG-immunized *cd73*^{*-*/*-*} and wild type mice displayed similar degrees of *in vitro* proliferation in response to varying concentrations of MOG peptide. However, CD4⁺ T cells from MOG-immunized *cd73*^{*-*/*-*} mice secreted higher levels of IL-17 and IL-1β following *in vitro* MOG stimulation, compared to those of wild type CD73⁺CD4⁺ or CD73⁻CD4⁺ T cells (Fig. 2C). Elevated levels of IL-17 are associated with MS (Matusevicius et al., "Interleukin-17 mRNA Expression in Blood and CSF Mononuclear Cells is Augmented in Multiple Sclerosis," Mult. Scler. 5:101-104 (1999)) and EAE development (Komiyama et al., "IL-17 Plays an Important Role in the Development of Experimental Autoimmune Encephalomyelitis," J. Immunol. 177:566-573 (2006)), while high levels of the proinflammatory IL-1β cytokine are a risk factor for MS (de Jong et al., "Production of IL-1beta and IL-1Ra as Risk Factors for Susceptibility and Progression of Relapse-Onset Multiple Sclerosis," J. Neuroimmunol. 126:172-179 (2002)) and an enhancer of IL-17 production (Sutton et al., "A Crucial Role for Interleukin (IL)-1 in the Induction of IL-17-Producing T Cells That Mediate Autoimmune Encephalomyelitis," J. Exp. Med. 203:1685-1691 (2006)). No difference in IL-2, IL-4, IL-5, IL-10, IL-13, INF-γ and TNF-α secretion was observed between wild type and *cd73*^{*-*/*-*} T cells following MOG stimulation (Fig. 2C). Overall, the results from these assays suggest that *cd73*^{*-*/*-*} T cells can respond well to MOG immunization.

It was then determined whether T cells from *cd73*^{*-*/*-*} mice possess the ability to cause EAE. To test this, CD4⁺ T cells from the spleen and lymph nodes of MOG immunized *cd73*^{*-*/*-*} mice were evaluated for their ability to induce EAE after transfer into *tcrα*^{*-*/*-*} (*cd73*^{+/+}) recipient mice. *Tcrα*^{*-*/*-*} mice lack endogenous T cells and cannot develop EAE on their own (Elliott et al., "Mice Lacking Alpha Beta + T Cells are Resistant to the Induction of Experimental Autoimmune Encephalomyelitis," J. Neuroimmunol. 70:139-144 (1996)). *Cd73*^{+/+}*tcrα^{-l-}* recipient mice that received CD4⁺ T cells from *cd73*^{*-*/*-*} donors developed markedly more severe disease compared to those that received wild type CD4⁺ T cells (Fig. 2D). Wild type and *cd73*^{*-*/*-*} donor CD4⁺ T cells displayed equal degrees of expansion following transfer into *cd73*^{*+*/}*⁺tcra*^{*-*/*-*} recipient mice. Thus, CD4⁺ T cells from *cd73*^{*-*/*-*} mice are not only capable of inducing EAE, but cause more severe EAE than those derived from wild type mice when transferred into *cd73*^{+/+}*tcrα*^{*-*/*-*} mice. These results are consistent with *in vitro* assays in which *cd73*^{*-*/*-*} CD4⁺ T cells secreted elevated levels of IL-17 and IL-1β (which are known to exacerbate EAE) in response to MOG stimulation (Fig. 2C) and suggest that *cd73*^{*-*/*-*} mice are resistant to MOG-induced EAE because of a lack of CD73 expression in non-hematopoietic cells (most likely lack of expression in the CNS).

### Example 11 - Cd73^{-/-} Mice Exhibit Little/No Lymphocyte Infiltration into the CNS Following EAE Induction.

EAE is primarily a CD4⁺ T cell mediated disease (Montero et al., "Regulation of Experimental Autoimmune Encephalomyelitis by CD4+, CD25+ and CD8+ T Cells: Analysis Using Depleting Antibodies," J. Autoimmun. 23:1-7 (2004)) and during EAE progression, lymphocytes must first gain access into the CNS in order to mount their inflammatory response against CNS antigens, resulting in axonal demyelination and paralysis (Brown et al., "Time Course and Distribution of Inflammatory and Neurodegenerative Events Suggest Structural Bases for the Pathogenesis of Experimental Autoimmune Encephalomyelitis," J. Comp. Neurol. 502:236-260 (2007)). To determine if CNS lymphocyte infiltration is observed following EAE induction in *cd73*^{*-*/*-*} mice, brain and spinal cord sections were examined for the presence of CD4⁺ T cells and CD45⁺ cells by immunohistochemistry. *Cd73*^{*-*/*-*} mice displayed a dramatically lower frequency of CD4⁺ (Figs. 3D-G) and CD45⁺ (Fig. 4) lymphocytes in the brain and spinal cord compared to wild type mice (Figs. 3A-C, G) at day 13 post MOG immunization. Additionally, in lymphocyte tracking experiments where MOG-specific T cells from 2d2 TCR transgenic mice (Bettelli et al., "Myelin Oligodendrocyte Glycoprotein-Specific T Cell Receptor Transgenic Mice Develop Spontaneous Autoimmune Optic Neuritis," J. Exp. Med. 197:1073-1081 (2003)) were transferred into either wild type or *cd73*^{*-*/*-*} mice with concomitant EAE induction, the percentage of 2d2 cells in the CNS increased several fold with time in wild type recipient mice, but not at all in *cd73*^{*-*/*-*} recipients (Fig. 5). Overall, these results suggest that the observed protection against EAE induction in *cd73*^{*-*/*-*} mice is associated with considerably reduced CNS lymphocyte infiltration. Nevertheless, CD4⁺ T cells from MOG-immunized *cd73*^{*-*/*-*} mice possessed the ability to gain access to the CNS when transferred into *cd73*^{+/+}*tcrα*^{*-*/*-*} mice that were concomitantly induced to develop EAE (Figs. 3K and 3L). In fact, earlier and more extensive CNS CD4⁺ lymphocyte infiltration was observed in *cd73*^{+/+}*tcrα*^{*-*/*-*} mice that received *cd73*^{*-*/*-*} CD4⁺ T cells (Figs. 3K,L) than in those that received wild type CD4⁺ T cells (Figs. 3H-J). Therefore, these results demonstrate that donor T cells from *cd73*^{*-*/*-*} mice have the ability to infiltrate the CNS of *cd73*^{+/+} recipient mice.

### Example 12 - CD73 Must be Expressed Either on Lymphocytes or in the CNS for Efficient EAE Development.

It was next asked whether CD73 expression on CD4⁺ T cells can compensate for a lack of CD73 expression in the CNS and allow the development of EAE. Therefore, CD4⁺ T cells were adoptively transferred from MOG-immunized wild type mice into *cd73*^{*-*/*-*} recipients, concomitantly induced EAE, and compared disease activity with that of similarly treated wild type recipients (Fig. 6A). While wild type recipients developed disease following EAE induction as expected, *cd73*^{*-*/*-*} recipients also developed prominent EAE with an average disease score of 1.5 by three weeks after disease induction. This was much higher than the 0.5 average score that *cd73*^{*-*/*-*} mice normally showed at this same time point (Fig. 1). To further define the association of CD4⁺ T cell CD73 expression with EAE susceptibility, sorted CD73⁺CD4⁺ and CD73⁻CD4⁺ T cells from immunized wild type mice, or total CD4⁺ (CD73⁻) T cells from immunized *cd73*^{*-*/*-*} mice, were transferred into *cd73*^{*-*/*-*} recipients with concomitant EAE induction (Fig. 6B). *Cd73*^{*-*/*-*} mice that received CD73⁺CD4⁺ T cells from wild type mice developed EAE with an average score of approximately 1.5 at three weeks post induction. Conversely, *cd73*^{*-*/*-*} mice that received wild type derived CD73⁻CD4⁺ T cells did not develop significant EAE. Additionally, CD4⁺ cells from *cd73*^{*-*/*-*} donor mice, which have the ability to cause severe EAE in CD73-expressing *tcrα*^{*-*/*-*} mice (Fig. 2D), were also incapable of potentiating EAE in recipient *cd73*^{*-*/*-*} mice (Fig. 6B). Therefore, although CD73 expression on T cells can partially compensate for a lack of CD73 expression in non-hematopoietic cells, EAE is most efficiently induced when CD73 is expressed in both compartments.

The identity of the CD73-expressing non-hematopoietic cells that promote the development of EAE is not known. Vascular endothelial cells at the BBB were considered as likely candidates, as many types of endothelial cells express CD73 (Yamashita et al., "CD73 Expression and Fyn-Dependent Signaling on Murine Lymphocytes," Eur. J. Immunol. 28:2981-2990 (1998)). However, immunohistochemistry revealed that mouse brain endothelial cells are CD73⁻. During these experiments, it was observed that CD73 is, however, highly expressed in the brain on the choroid plexus (Fig. 6C), which is an entry point into the CNS for lymphocytes during EAE progression (Brown et al., "Time Course and Distribution of Inflammatory and Neurodegenerative Events Suggest Structural Bases for the Pathogenesis of Experimental Autoimmune Encephalomyelitis," J. Comp. Neurol. 502:236-260 (2007)). Figure 4D shows infiltrating lymphocytes in association with the choroid plexus of wild type mice 12 days post-EAE induction. Minimal CD73 staining was also observed on submeningeal regions of the spinal cord. Taken together, these results suggest that CD73 expression, whether on T cells or in the CNS (perhaps on the choroid plexus), is necessary for efficient EAE development.

### Example 13 - Adenosine Receptor Antagonists Protect Mice Against EAE Induction.

As CD73 catalyzes the breakdown of AMP to adenosine and ARs are expressed in the CNS (Tsutsui et al., "A1 Adenosine Receptor Upregulation and Activation Attenuates Neuroinflammation and Demyelination in a Model of Multiple Sclerosis," J. Neurosci. 24:1521-1529 (2004)); Rosi et al., The Influence of Brain Inflammation Upon Neuronal Adenosine A2B Receptors," J. Neurochem. 86:220-227 (2003)), it was next determined if AR signaling is important during EAE progression. Wild type and *cd73*^{*-*/*-*} mice were treated with the broad spectrum AR antagonist caffeine (Dall'Igna et al., "Caffeine as a Neuroprotective Adenosine Receptor Antagonist," Ann. Pharmacother. 38:717-718 (2004)) at 0.6 g/L in their drinking water, which corresponds to an approximate dose of 4.0 mg/mouse of caffeine per day (Johansson et al., "A1 and A2A Adenosine Receptors and A1 mRNA in Mouse Brain: Effect of Long-Term Caffeine Treatment," Brain Res. 762:153-164 (1997)), 1 day prior to and throughout the duration of the EAE experiment (Fig. 7A). Wild type mice that received caffeine were dramatically protected against EAE development (Fig. 7A), comparable to previously published results (Tsutsui et al., "A1 Adenosine Receptor Upregulation and Activation Attenuates Neuroinflammation and Demyelination in a Model of Multiple Sclerosis," J. Neurosci. 24:1521-1529 (2004)). As expected, *cd73*^{*-*/*-*} mice that received caffeine did not develop EAE (Fig. 7A). Since CD73 is highly expressed on the choroid plexus (Fig. 6C), it was next determined if ARs are also expressed on the choroid plexus. Utilizing the Z310 murine choroid plexus cell line (Zheng et al., "Establishment and Characterization of an Immortalized Z310 Choroidal Epithelial Cell Line from Murine Choroid Plexus," Brain Res. 958:371-380 (2002)), only mRNA for the A₁ and A₂ₐ adenosine receptor subtypes were detected by qPCR (Fig. 7B). As *A₁AK*^{*-*/*-*} mice have been previously shown to develop severe EAE following disease induction (Tsutsui et al., "A1 Adenosine Receptor Upregulation and Activation Attenuates Neuroinflammation and Demyelination in a Model of Multiple Sclerosis," J. Neurosci. 24:1521-1529 (2004)), it was asked if treatment of wild type mice with SCH58261 (Melani et al., "The Selective A2A Receptor Antagonist SCH 58261 Protects From Neurological Deficit, Brain Damage and Activation of p38 MAPK in Rat Focal Cerebral Ischemia," Brain Res. 1073-1074:470-480 (2006)), an AR antagonist specific for the A₂ₐ subtype, could protect against EAE development. Wild type mice were given 1mg/kg of SCH58261 in DMSO or DMSO alone both *i.p.* and *s.c.* (for a total of 2mg/kg) 1 day prior to EAE induction and daily for 30 days throughout the course of the experiment (Fig. 7C). Wild type mice that received SCH58261 were dramatically protected against EAE development compared to wild type mice that received DMSO alone (Fig. 7C). Additionally, wild type mice given SCH58261 displayed a significantly lower frequency of CD4⁺ lymphocytes in the brain and spinal cord compared to DMSO treated wild type mice at day 15 post-EAE induction (Fig. 7D). As studies have shown that adhesion molecules (such as ICAM-1, VCAM-1, and MadCAM-1) on the choroid plexus play a role in the pathogenesis of EAE (Engelhardt et al., "Involvement of the Choroid Plexus in Central Nervous System Inflammation," Microsc. Res. Tech. 52:112-129 (2001)), it was determined if SCH58261 treatment affected adhesion molecule expression on the choroid plexus following EAE induction. Comparison of the choroid plexus from DMSO and SCH58261 treated wild type mice shows that A₂ₐ AR blockade prevented the up regulation of ICAM-1 that normally occurs during EAE progression (Fig. 8).

Based on these results, it was concluded that the inability of *cd73*^{*-*/*-*} mice to catalyze the generation of extracellular adenosine explains their failure to efficiently develop EAE following MOG immunization and that CD73 expression and A₂ₐAR signaling at the choroid plexus are requirements for EAE progression.

The goal of this study was to evaluate the role of CD73 in EAE, an animal model for MS. As CD73 catalyzes the formation of extracellular adenosine which is usually immunosuppressive (Bours et al., "Adenosine 5'-Triphosphate and Adenosine as Endogenous Signaling Molecules in Immunity and Inflammation," Pharmacol. Ther. 112:358-404 (2006)) and *A₁AR*^{*-*/*-*} mice exhibit severe EAE (Tsutsui et al., "A1 Adenosine Receptor Upregulation and Activation Attenuates Neuroinflammation and Demyelination in a Model of Multiple Sclerosis," J. Neurosci. 24:1521-1529 (2004)), applicants predicted that *cd73*^{*-*/*-*} mice would also develop severe EAE. However, *cd73*^{*-*/*-*} mice were highly resistant to EAE induction, a surprising finding considering the plethora of studies demonstrating that *cd73*^{*-*/*-*} mice are more prone to inflammation. For example, *cd73*^{*-*/*-*} mice are more susceptible to bleomycin-induced lung injury (Volmer et al., "Ecto-5'-Nucleotidase (CD73)-Mediated Adenosine Production is Tissue Protective in a Model of Bleomycin-Induced Lung Injury," J. Immunol. 176:4449-4458 (2006)) and are more prone to vascular inflammation and neointima formation (Zernecke et al., "CD73/ecto-5'-Nucleotidase Protects Against Vascular Inflammation and Neointima Formation," Circulation 113:2120-2127 (2006)). Consistent with these reports, applicants showed that *cd73*^{*-*/*-*} T cells produced higher levels of the EAE-associated proinflammatory cytokines IL-1β and IL-17 following MOG stimulation. Furthermore, the adoptive transfer of *cd73*^{*-*/*-*} T cells to *tcrα*^{*-*/*-*} mice, which lack T cells but express CD73 throughout their periphery, resulted in severe CNS inflammation following MOG immunization, consistent with a role for adenosine as an anti-inflammatory mediator. It is interesting to note that IFN-β treatment, one of the most effective therapies for MS, has been shown to up regulate CD73 expression on endothelial cells both *in vitro* and *in vivo* (Airas et al., "Mechanism of Action of IFN-Beta in the Treatment of Multiple Sclerosis: A Special Reference to CD73 and Adenosine," Ann. N. Y. Acad. Sci. 1110:641-648 (2007)). Thus, although the mechanism by which IFN-β benefits MS patients is incompletely understood, increased production of adenosine accompanied by its known anti-inflammatory and neuroprotective effects could be a factor.

Consistent with their resistance to EAE induction, *cd73*^{*-*/*-*} mice had a lower frequency of cells infiltrating the CNS during EAE compared to wild type mice. This was also an unexpected finding, as CD73-generated adenosine has previously been shown to restrict the migration of neutrophils across vascular endothelium during hypoxia and of lymphocytes across high endothelial venules of draining lymph nodes (Thompson et al., "Crucial Role for Ecto-5'-Nucleotidase (CD73) in Vascular Leakage During Hypoxia," J. Exp. Med. 200:1395-1405 (2004)). Applicants' data, in contrast, suggest that CD73, and the extracellular adenosine generated by CD73, are needed for the efficient passage of pathogenic T cells into the CNS. Therefore, the role that CD73 and adenosine play in CNS lymphocyte infiltration during EAE is more profound than their role in modulation of neuroinflammation.

It is important to know where CD73 must be expressed for T cell migration into the CNS. CD73 is found on subsets of T cells (Yamashita et al., "CD73 Expression and Fyn-Dependent Signaling on Murine Lymphocytes," Eur. J. Immunol. 28:2981-2990 (1998)) as well as on some epithelial (Strohmeier et al., "Surface Expression, Polarization, and Functional Significance of CD73 in Human Intestinal Epithelia," J. Clin. Invest. 99:2588-2601 (1997)) and endothelial cells (Yamashita et al., "CD73 Expression and Fyn-Dependent Signaling on Murine Lymphocytes," Eur. J. Immunol. 28:2981-2990 (1998)). The data presented here clearly demonstrates that although *cd73*^{*-*/*-*} T cells respond well to MOG immunization, they cannot enter the CNS unless CD73 is expressed in non-hematopoietic tissues (i.e. *cd73*^{+/+}*tcrα^{-l-}* mice which develop EAE after adoptive transfer of CD4⁺ T cells from *cd73*^{*-*/*-*} mice). A lack of CD73 on non-hematopoietic cells can also be compensated for, in part, by CD73 expression on T cells (i.e., *cd73*^{*-*/*-*} mice become susceptible to EAE when CD73⁺, but not CD73⁻, CD4⁺ T cells are adoptively transferred). While BBB endothelial cells as a relevant source of CD73 in the CNS were considered, because CD73 is expressed on human brain endothelial cells (Airas et al., "Mechanism of Action of IFN-Beta in the Treatment of Multiple Sclerosis: A Special Reference to CD73 and Adenosine," Ann. N. Y. Acad. Sci. 1110:641-648 (2007)), immunohistochemistry revealed that mouse brain endothelial cells are CD73⁻. However, CD73 was found to be highly expressed on choroid plexus epithelial cells, which form the barrier between the blood and the cerebrospinal fluid (CSF) and have a role in regulating lymphocyte immunosurveillance in the CNS (Steffen et al., "CAM-1, VCAM-1, and MAdCAM-1 are Expressed on Choroid Plexus Epithelium but Not Endothelium and Mediate Binding of Lymphocytes In Vitro," Am. J. Pathol. 148:1819-1838 (1996)). The choroid plexus has also been suggested to be the entry point for T cells during the initiation of EAE progression (Brown et al., "Time Course and Distribution of Inflammatory and Neurodegenerative Events Suggest Structural Bases for the Pathogenesis of Experimental Autoimmune Encephalomyelitis," J. Comp. Neurol. 502:236-260 (2007)). While the role of lymphocyte-brain endothelial cell interactions via VLA-4/VCAM-1 binding in both EAE and MS is well-documented (Rice et al., "Anti-Alpha4 Integrin Therapy for Multiple Sclerosis: Mechanisms and Rationale," Neurology 64:1336-1342 (2005)), perhaps lymphocyte trafficking across the endothelial BBB is more important for disease maintenance and progression than for disease initiation, at least in EAE.

The next issue is how CD73 facilitates the migration of T cells into the CNS. Earlier work showed that lymphocyte CD73 can promote the binding of human lymphocytes to endothelial cells in an LFA-1-dependent fashion (Airas et al., "CD73 Engagement Promotes Lymphocyte Binding to Endothelial Cells Via a Lymphocyte Function-Associated Antigen-1-dependent Mechanism," J. Immunol. 165:5411-5417 (2000)). This does not appear to be the function of CD73 in EAE, however, because CD73-deficient T cells can enter the CNS and cause severe disease in *cd73*^{+/+}*tcrα*^{*-*/*-*} mice (Fig. 2D). Alternatively, CD73 can function as an enzyme to produce extracellular adenosine, a ligand for cell surface ARs. It is this latter function that is relevant for the current work given that AR blockade with caffeine or SCH58261 can protect mice from EAE. While the broad spectrum AR antagonist caffeine also inhibits certain phosphodiesterases (Choi et al., "Caffeine and Theophylline Analogues: Correlation of Behavioral Effects With Activity as Adenosine Receptor Antagonists and as Phosphodiesterase Inhibitors," Life Sci. 43:387-398 (1988)), its modulation of EAE progression is most likely through its effect on AR signaling (Tsutsui et al., "A1 Adenosine Receptor Upregulation and Activation Attenuates Neuroinflammation and Demyelination in a Model of Multiple Sclerosis," J. Neurosci. 24:1521-1529 (2004)). This notion is supported by the fact that SCH58261 also prevents EAE progression by specifically inhibiting A₂ₐ AR signaling. As CD73 and the A₁ and A₂ₐ AR subtypes are expressed on the choroid plexus, extracellular adenosine produced by CD73 at the choroid plexus can signal in an autocrine fashion.

Since the A₁ and A₂ₐ ARs are functionally antagonistic to each other and have different affinities for adenosine (Quarta et al., "Opposite Modulatory Roles for Adenosine A1 and A2A Receptors on Glutamate and Dopamine Release in the Shell of the Nucleus Accumbens. Effects of Chronic Caffeine Exposure," J. Neurochem. 88:1151-1158 (2004)), the extracellular concentration of adenosine determines how a cell expressing the A₁ and A₂ₐ receptors will respond; thus creating a mechanistic switch whereby low concentrations of adenosine activate the A₁ subtype and higher concentrations stimulate the A₂ₐ subtype (Ciruela et al., "Presynaptic Control of Striatal Glutamatergic Neurotransmission by Adenosine A1-A2A Receptor Heteromers," J. Neurosci. 26:2080-2087 (2006)). In the CNS, there is evidence to suggest that this A₁/A₂ₐ interaction is important in mediating neuroinflammation, where A₁ signaling is protective while A₂ₐ signaling promotes inflammation. For example, mice that lack the A₁ adenosine receptor develop severe EAE following disease induction (Tsutsui et al., "A1 Adenosine Receptor Upregulation and Activation Attenuates Neuroinflammation and Demyelination in a Model of Multiple Sclerosis," J. Neurosci. 24:1521-1529 (2004)), while mice that are given an A₂ₐ antagonist are completely protected against EAE (Fig. 5C). Additionally, mice that lack the A₂ₐ receptor are protected from brain injury induced by transient focal ischemia (Chen et al., "A(2A) Adenosine Receptor Deficiency Attenuates Brain Injury Induced by Transient Focal Ischemia in Mice," J. Neurosci. 19:9192-9200 (1999)). Therefore, CD73-generated adenosine signaling at the choroid plexus appears to play a very important role in modulating inflammation in the CNS.

This adenosine signaling most likely regulates the expression of adhesion molecules at the choroid plexus. Studies have shown that the up regulation of the adhesion molecules ICAM-1, VCAM-1, and MadCAM-1 at the choroid plexus are associated with EAE progression (Engelhardt et al., Involvement of the Choroid Plexus in Central Nervous System Inflammation," Microsc. Res. Tech. 52:112-129 (2001)). As mice treated with the A₂ₐ AR antagonist SCH58261 do not experience increased choroid plexus ICAM-1 expression (Fig. 8), as normally occurs following EAE induction (Engelhardt et al., "Involvement of the Choroid Plexus in Central Nervous System Inflammation," Microsc. Res. Tech. 52:112-129 (2001)), the present results suggest that A₂ₐ AR signaling increases ICAM-1 during EAE progression.

In summary, this data shows that CD73 plays a critical role in the progression of EAE. Mice that lack CD73 are protected from the degenerative symptoms and CNS inflammation that are associated with EAE induction. This is the first study to demonstrate a requirement for CD73 expression and AR signaling for the efficient entry of lymphocytes into the CNS during EAE. The data presented here may mark the first steps of a journey that will lead to new therapies for MS and other neuroinflammatory diseases.

### Example 14 - The BBB Can be Modulated Through Activation of the Adenosine Receptors

The objective of this experiment was to determine if the blood brain barrier could be modulated by activation of adenosine receptors. NECA is a non-selective adenosine receptor agonist, with similar affinities for A1, A2a and A3 adenosine receptors and a low affinity for the A2b adenosine receptor. In order to determine if activation of adenosine receptors would induce extravasation of Evans Blue dye across the blood brain barrier (BBB), mice were treated with: NECA, a non-selective adenosine receptor agonist (n=5, 100µl 0.01nM); SCH58261, an A2a adenosine receptor specific antagonist (n=5, 1mg/kg); pertussis toxin, an agent known to induce BBB leakiness and as such used as a positive control (n=7, 200µl); and, PBS as a vehicle control (n=5, 100µl). CD73^{*-*/*-*} mice, which lack the ability to produce extracellular adenosine, were also treated with NECA (n=4, 100µl 0.01nM). Treatments were administered as a single i.v. injection one hour prior to i.v. injection of 200µl 1% Evans Blue dye (2µg total dye injected). Four hours after administration of Evans Blue, mice were anesthetized with a ketamine/xylazine mix and perfused via the left ventricle with ice cold PBS. Brains were harvested and homogenized in n,n-dimethylformamide (DMF) at 5µl/mg (v:w). Tissue was incubated for 72 hours at room temperature in DMF to extract the dye. Following extraction, the tissue / solvent mixture was centrifuged at 500xg for 30 minutes and 100µl of supernatant was read on a BioTex spectrophotometer at 620nm. Data is expressed as µg Evans Blue / ml DMF.

Treating mice with the general adenosine receptor agonist NECA can induce migration of dye across the blood brain barrier. This suggests that this barrier can be modulated through activation of the adenosine receptors. In Figure 9A, CD73^{*-*/*-*} mice, which lack extracellular adenosine and thus cannot adequately signal through adenosine receptors, were treated with NECA, resulting in an almost five fold increase in dye migration vs. the PBS control. SCH58261 was used as a negative control since applicants have shown that blocking of the A2a adenosine receptor using this antagonist can prevent lymphocyte entry into the brain (Mills et al., "CD73 is Required for Efficient Entry of Lymphocytes into the Central Nervous System During Experimental Autoimmune Encephalomyelitis," Proc. Natl. Acad. Sci. 105(27):9325-9330 (2008)). In Figure 9B, WT mice treated with NECA also show an increase over control mice. Pertussis is used as a positive control, as it is known to induce blood brain barrier leakiness in the mouse EAE model.

### Example 15 - The A2a and A2b Adenosine Receptors are Expressed on the Human Endothelial Cell Line hCMEC/D3

In order to establish an *in vitro* blood brain barrier (BBB), the human brain endothelial cell line hCMEC/D3 (Weksler et al., "Blood-brain Barrier-specific Properties of a Human Adult Brain Endothelial Cell Line," J. Neurochem. 19(13):1872-4 (2005); Poller et al., "The Human Brain Endothelial Cell Line hCMEC/D3 as a Human Blood-brain Barrier Model for Drug Transport Studies," J. Neurochem. 107(5):1358-1368 (2008)) was obtained, which has been previously described as having BBB properties. Here, expression pattern of adenosine receptors on these cells was established.

hCMEC/D3 cells were grown to confluence, harvested and RNA was extracted using TRIzol reagent (Invitrogen, Carlsbad, CA) according to the manufacturer's instructions. cDNA was synthesized using a Verso cDNA kit (Thermo Scientific, Waltham, MA), and Real Time PCR was performed using Power SYBR Green (Applied Biosystems, Foster City, CA).

As shown in Figure 10, the A2a and A2b adenosine receptors were found to be expressed on the human endothelial cell line hCMEC/D3.

### Example 16 - Adenosine Receptor Stimulation of Brain Endothelial Cells Promotes Lymphocyte Migration Through the BBB

The blood brain barrier (BBB) is comprised of endothelial cells. During late stages of EAE, lymphocytes are known to cross the BBB. In order to determine if adenosine receptor stimulation of brain endothelial cells could promote lymphocyte migration through the BBB, an *in vitro* BBB was established. The human brain endothelial cell line hCMEC/D3 (Weksler et al., "Blood-brain Barrier-specific Properties of a Human Adult Brain Endothelial Cell Line," J. Neurochem. 19(13):1872-4 (2005); Poller et al., "The Human Brain Endothelial Cell Line hCMEC/D3 as a Human Blood-brain Barrier Model for Drug Transport Studies," J. Neurochem. 107(5):1358-1368 (2008)) was obtained, which has been previously described as having BBB properties.

hCMEC/D3 cells were seeded onto Transwell and allowed to grow to confluencey. 2x10⁶ Jurkat cells were added to the upper chamber with or without NECA (general adenosine receptor [AR] agonist), CCPA (A1AR agonist), CGS 21680 (A2AAR agonist), or DMSO vehicle. After 24 hours, migrated cells in the lower chamber were counted. Values are relative to the number of cells that migrate through non-HCMECD3 seeded transwells.

As shown in Figure 11, NECA, a broad spectrum adenosine receptor agonist, induced some migration. CGS, the A2a adenosine receptor agonist, promoted lymphocyte migration across the *in vitro* BBB when used at a lower concentration. CCPA, the A1 agonist, induced lymphocyte migration at high levels possibly due to activation of the A2a adenosine receptor, which has a lower affinity for CCPA and thus is only activated at higher levels of CCPA.

### Example 17- A2a Adenosine Receptor Activation Promotes Lymphocyte Migration Across the CP

The choroid plexus ("CP") controls lymphocyte migration into the CNS. The CP expresses the A1 and A2a adenosine receptors. EAE is prevented in mice when A2a adenosine receptor activity is blocked. EAE is enhanced when the A1 adenosine receptor is missing. It was hypothesized that A2a adenosine receptor activation promotes lymphocyte migration across the CP. Z310 cells are a murine choroid plexus cell line.

To test the hypothesis, Transwell membranes were seeded with Z310 cells and allowed to grow to confluencey. 2x10⁶ Jurkat cells were added to the upper chamber with or with out NECA (n=1, general AR agonist), CCPA (n=1, A1AR agonist), CGS 21680 (n=1, A2AAR agonist), or DMSO vehicle(n=1). After 24 hours, migrated cells in the lower chamber were counted. Values are relative to the number of cells that migrate through non-Z310 seeded transwells and the results are shown in Fig. 12.

As shown in Fig. 12, NECA, a broad spectrum adenosine receptor agonist, induced migration. CGS, the A2a adenosine receptor agonist, promoted lymphocyte migration across the CP. CCPA, the A1 agonist, induced lymphocyte migration at high levels possibly due to activation of the A2a adenosine receptor, which has a lower affinity for CCPA and as such is only activated at high levels of CCPA.

### Example 18 - Human Brain Endothelial Cells are Sensitive to Adenosine Receptor Induced cAMP Regulation

Adenosine receptor activation regulates cAMP levels in cells. In order to determine the sensitivity of human brain endothelial cells to adenosine receptor induced cAMP regulation, human brain endothelial cells were cultured with adenosine receptor agonists at various concentrations, followed by cAMP level analysis, as shown in Fig. 13.

HCMECD3 cells were grown to confluencey on 24 well plates. As adenosine receptor ("AR") stimulation is known to influence cAMP levels, cells were treated with or without various concentrations of NECA (general AR agonist), CCPA (A1AR agonist), CGS 21680 (A2AAR agonist), DMSO vehicle, or Forksolin (induces cAMP). After 15 minutes, lysis buffer was added and the cells were frozen at -80C to stop the reaction. Duplicate samples were used for each condition. cAMP levels were assayed using a cAMP Screen kit (Applied Biosystems, Foster City, CA).

As shown in Fig. 13, the broad spectrum adenosine receptor agonist NECA increased cAMP levels, verifying that these cells can respond to adenosine receptor signaling. High levels of CCPA, the A1 adenosine receptor agonist, increased cAMP levels, again perhaps due to activation of the A2a adenosine receptor, which has a lower affinity for CCPA and as such is only activated at high levels of CCPA. CGS, the A2a adenosine receptor agonist slightly increased cAMP levels in the human brain endothelial cell line.

### Example 19 - Female A1 Adenosine Receptor Knockout Mice Develop More Severe EAE Than Wild Type

A1 and A2a adenosine receptors are expressed on the choroid plexus. A2a adenosine receptor antagonists protect mice from EAE. Are mice that lack the A1 adenosine receptor prone to development of more severe EAE than wild type controls? To answer this question, disease profiles of wild type and A1 adenosine receptor null mice were compared.

Female A1 adenosine receptor knockout (A1ARKO, n=5) and wild type (WT, n=5) mice were immunized with CFA/MOG₃₅₋₅₅ + PTX on 12-2-08 and scored daily for 41 days. As the results in Fig. 14 illustrate, A1ARKO mice develop more severe EAE than WT, and also develop disease at a faster rate than WT.

### Example 20 - Brains From Wild Type Mice Fed an Adenosine Receptor Antagonist Have Higher Levels of FITC-Dextran Than Brains from CD73^{-/-} Mice Fed an Adenosine Receptor Antagonist

In order to examine the effects of caffeine, a general adenosine receptor antagonist, on blood brain barrier permeability, mice were fed caffeine for several days and then injected with FITC Dextran, commonly used to assess endothelial permeability.

More particularly, mice were fed 0.6g/l caffeine (Sigma, St. Louis, MO) in water or regular water ad lib for five days. Mice were injected IP with FITC Dextran (10,000 MW, Molecular Probes, Eugene, OR) and after 30 minutes mice were perfused with ice cold PBS via the left ventricle. Brains were removed and snap frozen in OCT (Tissue Tek, Torrance, CA) and stored at -80°C until sectioning. Tissue sections (5µm) were stained with hematoxylin for light microscopy and with DAPI for a fluorescent counterstain. The results are shown in Fig. 15.

As shown in Figure 15A, visualization of brain sections from CD73^{-/-} mice fed caffeine displayed a much less intense green color than wild type mice, indicating less FITC-Dextran extravasation across the blood brain barrier. Brain sections from wild type mice displayed an intensely green background (Figure 15B) that is indicative of more FITC-dextran extravasation across the blood brain barrier. Figure 16 shows the results for wild-type mice in graphical form.

### Example 21- Adenosine Receptor Agonist NECA Increases Evans Blue Dye Extravasation Across the Blood Brain Barrier

The objective of this experiment was to determine if the blood brain barrier could be modulated by activation of adenosine receptors. NECA is a non-selective adenosine receptor agonist, with similar affinities for A1, A2A and A3 adenosine receptors and a low affinity for the A2b adenosine receptor.

In order to determine if activation of adenosine receptors would induce extravazation of Evans Blue dye across the blood brain barrier (BBB), mice were first treated on day one with NECA, a non-selective adenosine receptor agonist (n=2, 100µl 0.01nM); and, PBS as a vehicle control (n=2, 100µl). On day 2 mice were then immunized with CFA-MOG₃₅₋₅₅ and pertussis to induce EAE. Then NECA or PBS was administered every other day on day 3, day 5, day 7 and day 9. On day 10, mice were injected intravenously with 200µl 1% Evans Blue dye (2µg total dye injected). Six hours after administration of Evans Blue, mice were anesthetized with a ketamine/xylazine mix and perfused via the left ventricle with ice cold PBS. Brains were harvested and homogenized in n,n-dimethylformamide (DMF) at 5µl/mg (v:w). Tissue was incubated for 72 hours at room temperature in DMF to extract the dye. Following extraction, the tissue / solvent mixture was centrifuged at 500xg for 30 minutes and 100µl of supernatant was read on a BioTex spectrophotometer at 620nm. Data is expressed as pg Evans Blue / ml DMF and is shown in Figure 17.

This experiment demonstrates that treatment of mice with the general adenosine receptor agonist NECA induces migration of Evans Blue dye into the CNS in mice immunized for EAE. This suggests that the blood brain barrier in the EAE model can be modulated through activation of the adenosine receptors. WT EAE mice treated with NECA show an increase in BBB permeability over PBS control EAE mice.

## Claims

1. An A2A adenosine receptor agonist and/or an A1 adenosine receptor antagonist, for use in increasing blood brain barrier permeability in a subject, wherein the subject is one who would benefit from increased blood brain barrier permeability and wherein the subject is not subjected to a treatment which increases adenosine level.

2. The agent of claim 1, wherein the agent is an A2A adenosine receptor agonist.

3. The agent of claim 1, wherein the agent is an A1 adenosine receptor antagonist.

4. An A2A adenosine receptor antagonist for use in decreasing blood brain barrier permeability in a subject, wherein the subject is one who would benefit from decreased blood brain barrier permeability.

5. The agent of claim 4, wherein the subject has an inflammatory disease, or wherein the subject has a condition mediated by entry of lymphocytes into the brain.

6. The agent of claim 4, wherein the subject has the condition multiple sclerosis.

7. Use of an A2A adenosine receptor agonist and/or an A1 adenosine receptor antagonist, in the manufacture of a medicament for increasing blood brain barrier permeability in a subject, wherein the subject is one who would benefit from increased blood brain barrier permeability and wherein the subject is not subjected to a treatment which increases adenosine level.

8. Use of an A2A adenosine receptor antagonist in the manufacture of a medicament for decreasing blood brain barrier permeability in a subject, wherein the subject is one who would benefit from decreased blood brain barrier permeability.

## Patentansprüche

1. A2A-Adenosinrezeptoragonist und/oder A1-Adenosinrezeptorantagonist für den Gebrauch beim Erhöhen der Permeabilität der Blut-Hirn-Schranke in einem Subjekt, wobei das Subjekt eines ist, das von einer erhöhten Permeabilität der Blut-Hirn-Schranke Nutzen ziehen würde, und wobei das Subjekt nicht unter einer Behandlung steht, die den Adenosinspiegel steigert.

2. Mittel nach Anspruch 1, wobei das Mittel ein A2A-Adenosinrezeptoragonist ist.

3. Mittel nach Anspruch 1, wobei das Mittel ein A1-Adenosinrezeptorantagonist ist.

4. A2A-Adenosinrezeptorantagonist für den Gebrauch zum Verringern der Permeabilität der Blut-Hirn-Schranke in einem Subjekt, wobei das Subjekt eines ist, das von einer verringerten Permeabilität der Blut-Hirn-Schranke Nutzen ziehen würde.

5. Mittel nach Anspruch 4, wobei das Subjekt an einer Entzündungserkrankung leidet oder wobei das Subjekt an einer Krankheit leidet, die durch das Eindringen von Lymphozyten in das Gehirn vermittelt wird.

6. Mittel nach Anspruch 4, wobei das Subjekt unter der Krankheit multiple Sklerose leidet.

7. Gebrauch eines A2A-Adenosinrezeptoragonisten und/oder eines A1-Adenosinrezeptorantagonisten beim Herstellen eines Medikaments zum Erhöhen der Permeabilität der Blut-Hirn-Schranke in einem Subjekt, wobei das Subjekt eines ist, das von einer erhöhten Permeabilität der Blut-Hirn-Schranke Nutzen ziehen würde, und wobei das Subjekt nicht unter einer Behandlung steht, die den Adenosinspiegel steigert.

8. Gebrauch eines A2A-Adenosinrezeptorantagonisten beim Herstellen eines Medikaments zum Verringern der Permeabilität der Blut-Hirn-Schranke in einem Subjekt, wobei das Subjekt eines ist, das von einer verringerten Permeabilität der Blut-Hirn-Schranke Nutzen ziehen würde.

## Revendications

1. Agoniste du récepteur A2A de l'adénosine et/ou antagoniste du récepteur A1 de l'adénosine pour une utilisation dans l'augmentation de la perméabilité de la barrière hémato-encéphalique chez un patient, dans lequel le patient est un patient qui bénéficierait d'une perméabilité accrue de la barrière hémato-encéphalique et dans lequel le patient n'est pas soumis à un traitement qui augmente le taux d'adénosine.

2. Agent selon la revendication 1, lequel agent est un agoniste du récepteur A2A de l'adénosine.

3. Agent selon la revendication 1, lequel agent est un antagoniste du récepteur A1 de l'adénosine.

4. Agoniste du récepteur A2A de l'adénosine pour une utilisation dans la réduction de la perméabilité de la barrière hémato-encéphalique chez un patient, dans lequel le patient est un patient qui bénéficierait d'une perméabilité réduite de la barrière hémato-encéphalique.

5. Agent selon la revendication 4, dans lequel le patient a une maladie inflammatoire, ou dans lequel le patient a une pathologie médiée par l'entrée de lymphocytes dans le cerveau.

6. Agent selon la revendication 4, dans lequel le patient a une sclérose en plaques.

7. Utilisation d'un agoniste du récepteur A2A de l'adénosine et/ou d'un antagoniste du récepteur A1 de l'adénosine, dans la fabrication d'un médicament pour augmenter la perméabilité de la barrière hémato-encéphalique chez un patient, dans laquelle le patient est un patient qui bénéficierait d'une perméabilité accrue de la barrière hémato-encéphalique et dans laquelle le patient n'est pas soumis à un traitement qui augmente le taux d'adénosine.

8. Utilisation d'un antagoniste du récepteur A2A de l'adénosine dans la fabrication d'un médicament pour réduire la perméabilité de la barrière hémato-encéphalique chez un patient, dans laquelle le patient est un patient qui bénéficierait d'une perméabilité réduite de la barrière hémato-encéphalique.
